Europäisches Patentamt
European Patent Office
Office européen des brevets

Publication number: 0 275 002 A1

# EUROPEAN PATENT APPLICATION

Application number: 88100008.7

Date of filing: 04.01.88

Int. Cl.⁴: C07D 499/00 , //C07F9/65, C07F7/18

Priority: 09.01.87 GB 8700468
09.01.87 GB 8700469
09.01.87 GB 8700470
09.01.87 GB 8700471

Date of publication of application: 20.07.88 Bulletin 88/29

Designated Contracting States: AT BE CH DE ES FR GB GR IT LI LU NL SE

Applicant: HOECHST AKTIENGESELLSCHAFT
Postfach 80 03 20
D-6230 Frankfurt am Main 80(DE)

Inventor: Barker, Andrew John
152 Sutherland Grove West Bletchley
Milton Keynes Bucks.(GB)
Inventor: Campbell, Malcolm
Saffron House Holcombe Hill
Holcombe Bath Avon.(GB)
Inventor: Jenkins, Mark John
1 Isis Walk Bletchley
Milton Keynes Bucks. MK3 7DA(GB)

## Process for the production of 7-oxo-4-thia-1-azabicyclo [3.2.0] hept-2-ene derivatives.

A process for the production of a penem compound I

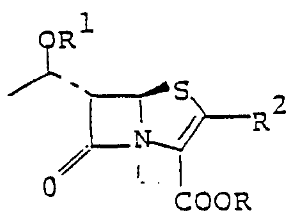

(I)

with R being H or an esterifying group, $R^1$ being H or a protecting group and $R^2$ being H or a great number of substituents
is disclosed with process comprises causing or allowing a compound II

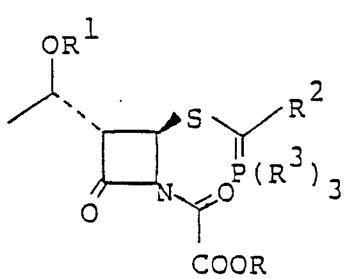

II

to cyclise.
Compound II is obtained according to the following reaction scheme.

EP 0 275 002 A1

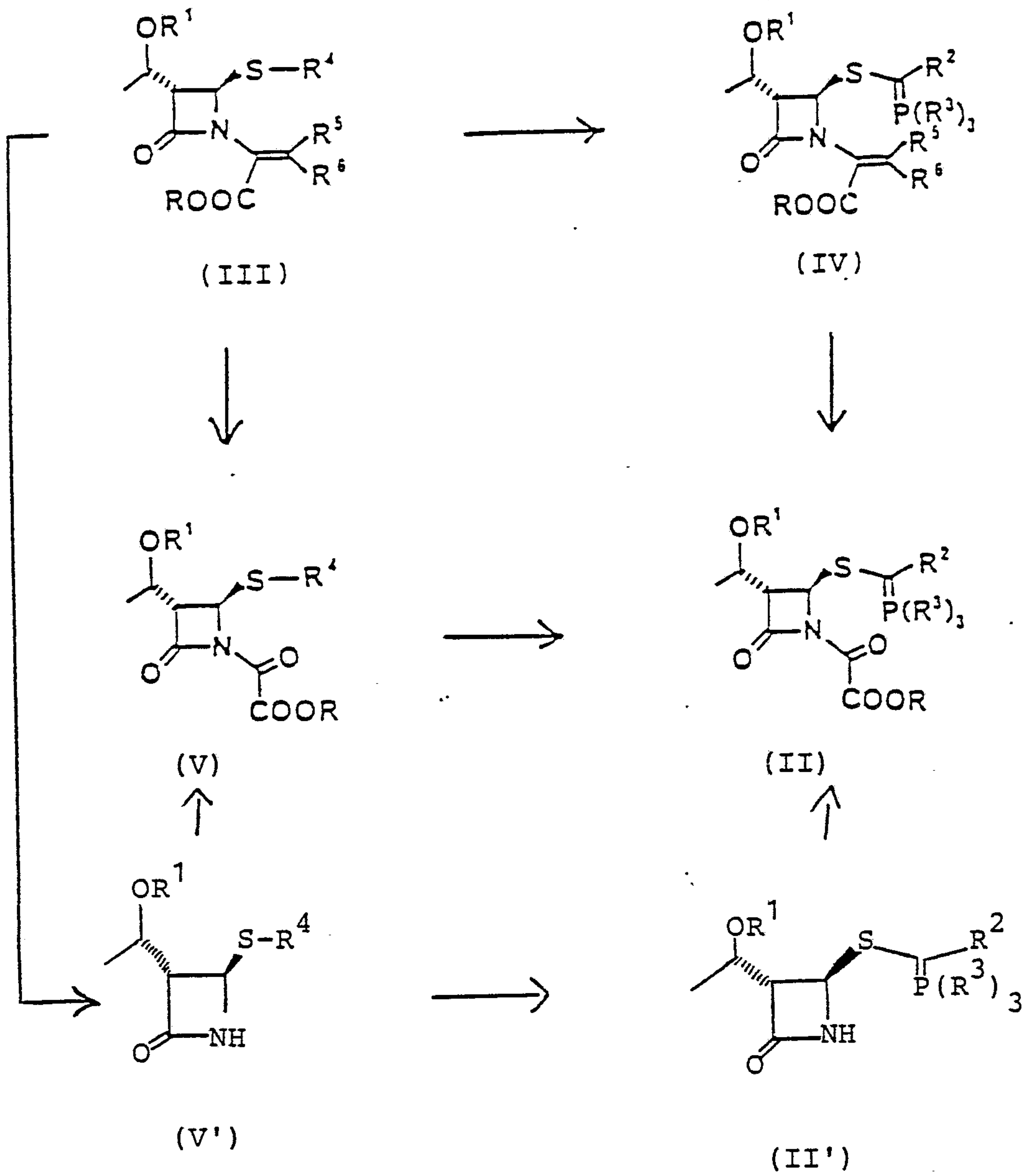

(III) (IV)

(V) (II)

(V') (II')

## Process for the production of 7-oxo-4-thia-1-azabicyclo[3.2.0]hept-2-ene derivatives

This invention relates to a process for the production of 7-oxo-4-thia-1-azabicyclo[3.2.0]hept-2-ene derivatives having anti-bacterial activity and/or having $\beta$-lactamase inhibitory and/or inactivating activity.

The penicillins as a class have in common the $\beta$-lactam structure A known semi-systematically as the "penam" nucleus. The introduction of a double bond between carbon atoms 2 and 3 in this structure gives rise to the "penem" nucleus B.

(A) 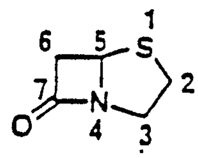 . 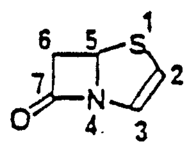 (B)

The penem nucleus B is given the systematic name "7-oxo-4-thia-1-azabicyclo[3.2.0]hept-2-ene", with the numbering as shown in formula C. A side chain at position 6 is numbered as shown in formula D.

(C) 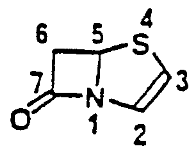 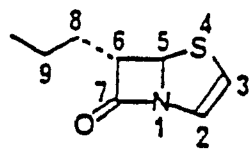 (D)

In the search for new types of $\beta$-lactam antibiotics penems have emerged as promising candidates. Although various processes for the production of penem derivatives have been described, there is still a need for processes that start from readily available and relatively inexpensive starting materials, and that involve mild reaction conditions and as few reaction steps as possible.

The present invention relates to a process for the production of penem derivatives under mild conditions from a compound that can itself be produced under mild conditions from commercially available penicillin derivatives.

The present invention provides a process for the production of a penem compound of formula I or a salt of a penem compound of formula I having a salt forming group

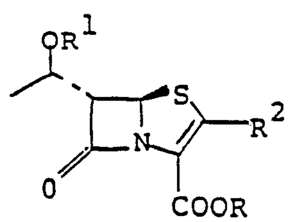 (I) .

wherein
R is hydrogen
$C_1$-$C_4$-alkyl, $C_1$-$C_4$-alkyloxycarbonyl-$C_1$-$C_4$-alkyl, $C_2$-$C_4$-alkenyl, that may be substituted by halogen, benzyl that is unsubstituted or is substituted by one or two substituents selected from methoxy, nitro groups and halogen atoms;
1′-(phenoxy)ethyl, 2,2,2-trichloroethyl, $C_1$-$C_4$-trialkylsilyl, $C_1$-$C_4$-trialkylsilyl-$C_1$-$C_4$-alkyl, phthalidyl, benzhydryl, trityl, acetonyl, $C_2$-$C_{12}$-acyloxymethyl, 1′-($C_2$-$C_{12}$-acyloxy) ethyl, amino-$C_2$-$C_{12}$-alkanoyloxymethyl, 1′-(amino-$C_2$-$C_{12}$-alkanoyloxy) ethyl, $C_2$-$C_{12}$-alkoxycarbonyloxymethyl, 1′-($C_2$-$C_{12}$-alkoxycarbonyloxy) ethyl, optionally substituted 2-aminoethyl,
$R^1$ is hydrogen,
tetrahydropyranyl, tetrahydrofuranyl,
$Si(Ra)_3$ in which the three groups Ra are the same or different and each represents a $C_1$-$C_4$-alkyl group or a phenyl group;
$CO_2R_b$, in which $R_b$ represents a p-nitrobenzyl, 2-trimethylsilylethyl, 2,2,2-trichloroethyl, benzyl or allyl group;
$COR_c$, in which $R_c$ represents hydrogen, a $C_1$-$C_6$-alkyl group, a phenyl or a phenoxy-$C_1$-$C_4$-alkyl group,

$R^2$ is
hydrogen, -CHO, CN
COOR, in which R is defined as above,
CO-$C_1$-$C_4$-alkyl, $C_1$-$C_4$-alkyl, $C_1$-$C_4$-alkyloxy, $C_1$-$C_4$-alkylthio, $C_1$-$C_4$-alkylsulphinyl, $C_1$-$C_4$-alkylsulfonyl, wherein the alkyl groups are unsubstituted or substituted by one or more substituents selected from F, Cl, BR, J atoms: optionally substituted $C_3$-$C_8$-cycloalkyl, phenyl or heterocyclyl; CN, $CO_2R$, $CONH_2$, CONH Rd, CONH-($C_1$-$C_4$-alkyl), OH, O($C_1$-$C_4$-alkyl), O($C_3$-$C_8$-cycloalkyl), O-phenyl, O-heterocyclyl, OR', OCO($C_1$-$C_4$-alkyl), $OCONH_2$, OCONH($C_1$-$C_4$-alkyl), -$OCONHR_D$, $S(O)_p$ ($C_1$-$C_4$-alkyl), $S(O)_p$($C_3$-$C_8$-cycloalkyl), $S(O)_p$phenyl, $S(O)_p$heterocyclyl, $SO_2NH_2$, $SO_2NHR_D$, $NH_2$, NH ($C_1$-$C_4$-alkyl), N($C_1$-$C_4$-alkyl$)_2$, NH-phenyl, $NHR_D$, optionally substituted amidino, aminomethylenamino or guanidino,
in which groups R and R' are defined as above, $R_D$ represents an amino protecting group and P represents 0, 1 or 2;
CO-phenyl, CO-heterocyclyl, phenyl, phenoxy, phenyl-$S(O)_p$, heterocyclyl, heterocyclyloxy, heterocyclyl-$S(O)_p$, wherein p is defined as above and the heterocycle is a mono-or bicyclic ring system, optionally unsaturated and containing one or more O, S or N atoms and which may be bound via a ring C or N atom and wherein
the phenyl or heterocyclyl group is unsubstituted or substituted by one or more substituents selected from halogen atoms, $C_1$-$C_4$-alkyl, $C_1$-$C_4$-alkoxy, optionally substituted $C_3$-$C_8$-cycloalkyl and $C_3$-$C_8$-cycloalkyloxy; $NO_2$, CN, CHO, $CO_2R$, CO($C_1$-$C_4$-alkyl), $CONH_2$, COHN ($C_1$-$C_4$-alkyl), $CONHR_D$, CONHOH, CONHO($C_1$-$C_4$-alkyl), CH = NOH, $CSNH_2$, C( = NOH)$CH_3$, $NH_2$, NH($C_1$-$C_4$-alkyl) $NHR_d$, NHCHO, NHCO($C_1$-$C_4$-alkyl), $S(O)_p$ ($C_1$-$C_4$-alkyl), $SO_2NH_2$, $SO_2NH$($C_1$-$C_4$-alkyl), $SO_2NHR_D$, $NHSO_2$($C_1$-$C_4$-alkyl), NHCONH($C_1$-$C_4$-alkyl), amidino, aminomethylenamino, guanidino, COX phenyl, in which X is oxygen or sulphur and the phenyl moiety is unsubstituted or substituted by one or more groups, selected from $C_1$-$C_4$-alkoxy, CN, $NO_2$ or halogen atoms, $CO_2Si(Ra)_3$ in which Ra is defined as above, $CONH(CH_2)_mQ$ or -NHCO $(CH_2)_mQ$ in which m represents an integer from 1 to 3, and Q represents one of the following groups:
CN, $SO_2NH_2$, $SO_2NHR_D$, $SOCH_3$, $SOC_2H_5$, $SO_2CH_3$, $SO_2C_2H_5$, $NH_2$, NHCH = NH, $CONH_2$, $CONHR_D$ $CONHCH_3$, CONHOH, NH($C_1$-$C_4$-alkyl), $NHR_D$, $OCONH_2$, NH-C($CH_3$) = NH;
$CONH_2$, CONH($C_1$-$C_4$-alkyl), CONH-phenyl, CH = $CH_2$, CH = CH($C_1$-$C_4$-alkyl), CH = CH-phenyl, CH = CHCO($C_1$-$C_4$-alkyl), CH = CHCO-phenyl or CH = $CHCO_2$($C_1$-$C_4$-alkyl); optionally substituted $C_3$-$C_8$-cycloalkyl, $C_3$-$C_8$-cycloalkyloxy and $C_3$-$C_8$-cycloalkyl $S(O)_p$, wherein p and $R_D$ are defined as above and the cycloalkyl group is saturated or contains a double bond and in which one ring carbon atom can be replaced by O, S or N, wherein the cycloalkyl group may be bound via a ring C or N atom and wherein the cycloalkyl group is unsubstituted or substituted as defined above for the alkyl moiety.

In general, preferred substituents for alkyl groups are halogen atoms such as fluorine and -COOR, -CN, -OH, -OCO($C_1$-$C_4$-alkyl), -$S(O)_p$($C_1$-$C_4$-alkyl), -$CONH_2$, -$NH_2$, -$OCONH_2$, $OCOCH_3$, -NHCHO, -$SO_2NH_2$, -$CONHCH_2CONH_2$, -$CONHCH_2CH_2NH_2$ and -NH-CH = NH groups, in which R, $R_d$and p are defined as above. For phenyl groups, preferred substitutents include -CN, -$CONH_2$, -$CONHCH_3$, -NHCHO, -$CONHCH_2CONH_2$ and -$CONHCH_2CH_2NH_2$ groups.

The present invention particularly relates to compounds in which R and $R_1$ have the above meanings and $R^2$ denotes hydrogen, CHO, CN,
$CO_2R$ such as, for example $CO_2H$, $CO_2CH_3$, $CO_2C_2H_5$,

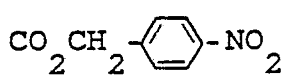,

$CO_2CH_2CCl_3$; $CO_2C(CH_3)_3$ CO-$C_1$-$C_4$-alkyl, such as, for example, $COCH_3$; optionally substituted straight-chained or branched $C_1$-$C_4$-alkyl, such as, for example, methyl, ethyl, propyl, isopropyl,
-$CH_2NH_2$, -$CH_2$-CH($CH_3$)-$NH_2$, -$CH_2OCONH_2$, -$CH_2OCOCH_3$; benzyl,

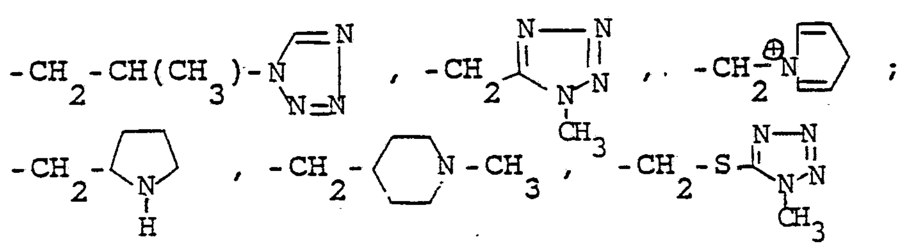

optionally substituted $C_1$-$C_4$-alkyloxy, such as, for example - $OCH_3$
optionally substituted $C_1$-$C_4$-alkylthio, such as, for example, methylthio, ethylthio, $SCH_2CH_2OCONH_2$, $S(CH_2)_2SO_2NH_2$, $SCH_2CH(NH_2)$ COOH, -S $CH_2CH_2$ COOH, -S-$CH_2CH_2$ OH, -$SCH_2CH_2F$, -$SCH_2CH_2NH_2$, $SCH_2CH_2CONHCH_3$,

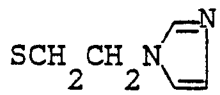

optionally substituted CO-phenyl, phenyl, CO heterocyclyl or heterocyclyl, such as, for example,

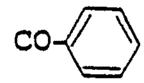 , 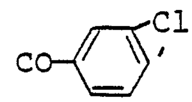 ,

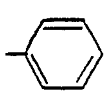 , 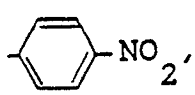 , 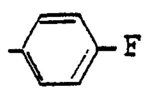 , 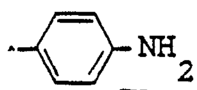 , 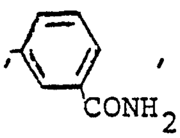 , 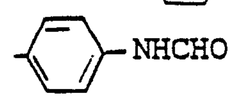 ,

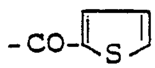 , 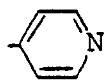 , 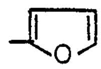 , 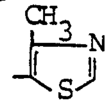 ;

pyrrolyl, pyrazolyl, imidazolyl, triazolyl, tetrazolyl, bound via a ring C or N atom;
optionally substituted phenyloxy or heterocyclyloxy, such as, for example,

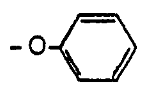 , 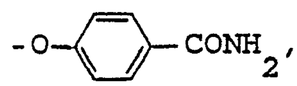 , 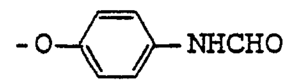 ,

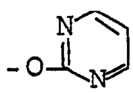 ;

optionally substituted phenylthio or heterocyclyl thio, such as

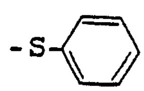 ; 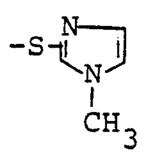

-$CONH_2$, $CH=CHC_6H_5$, optionally substituted $C_3$-$C_8$-cycloalkyl, $C_3$-$C_8$-cycloalkyloxy, $C_3$-$C_8$-cycloalkyl $S(O)_p$, such as, for example, tetrahydrofuranyl, tetrahydropyranyl, 1,4-dioxanyl,

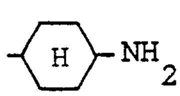 , 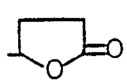 , 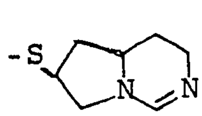 , 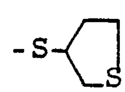 , 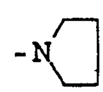

The process, according to the invention for the preparation of compounds of formula I comprises causing or allowing a compound for formula II

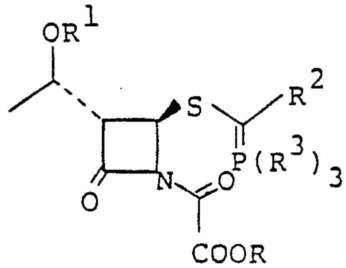

II

in which R, R$^1$ and R$^2$ are defined as above and
R$^3$ is $C_1$-$C_8$-alkyl, $C_3$-$C_8$-cycloalkyl or phenyl that is unsubstituted or is substituted by a methyl or methoxy group, the three groups R$^3$ being the same or different, to cyclise.

In most cases the cyclisation occurs spontaneously. It may be advantageous to perform the cyclisation under an inert atmosphere, for example, under nitrogen and/or in the presence of a free radical acceptor, for example, hydroquinone.

The cyclisation of compound II to give compound I is generally carried out in a solvent, for example, selected from chlorinated hydrocarbons, for example, chloroform and dichloromethane; ethers, for example, diethyl ether, 1,2-dimethyoxyethane and tetrahydrofuran; aromatic hydrocarbons, for example, benzene and toluene; aliphatic hydrocarbons, for example, hexane; esters, for example, ethyl acetate; acetonitrile and dimethylformamide. The cyclisation temperature is, for example, within the range of from -70°C to the boiling point of the solvent used, preferably from -20°C to room temperature or above.

As indicated above, cyclisation of compound II in most cases occurs spontaneously. Accordingly, compound II may be allowed to stand at room temperature or below or to warm from a temperature below room temperature to room temperature. It may be desired in some cases to heat compound II above room temperature, but in most cases this is not necessary.

Amino protecting groups are well known and are described, for example, in Protecting Groups in Organic Chemistry Ed. J.F.W. McOmie, Plenum Press, 1973 and Protective Groups in Organic Synthesis, T.W. Greene, J. Wiley & Sons Inc, 1981. Examples of amino protecting groups include alkoxycarbonyl groups having from 1 to 4 carbon atoms in the alkyl moiety and optionally one or more halogen atom substituents, for example, t-butyloxycarbonyl, 2-iodoethoxycarbonyl and 2,2,2-trichloroethoxycarbonyl groups; allyloxycarbonyl, benzyl and benzyloxycarbonyl groups that may be substituted, for example, by a methyl, methoxy or nitro group, for example, p-methoxybenzyl, p-methoxybenzyloxycarbonyl, p-nitrobenzyl and p-nitrobenzyloxycarbonyl groups; diphenylmethyl and trityl groups; and $Si(Ra)_3$ groups in which each group Ra is defined as above, for example, dimethyl-t-butylsilyl and trimethylsilyl groups.

A compound of formula II may be produced from a compound of formula III as shown in the following reaction scheme:

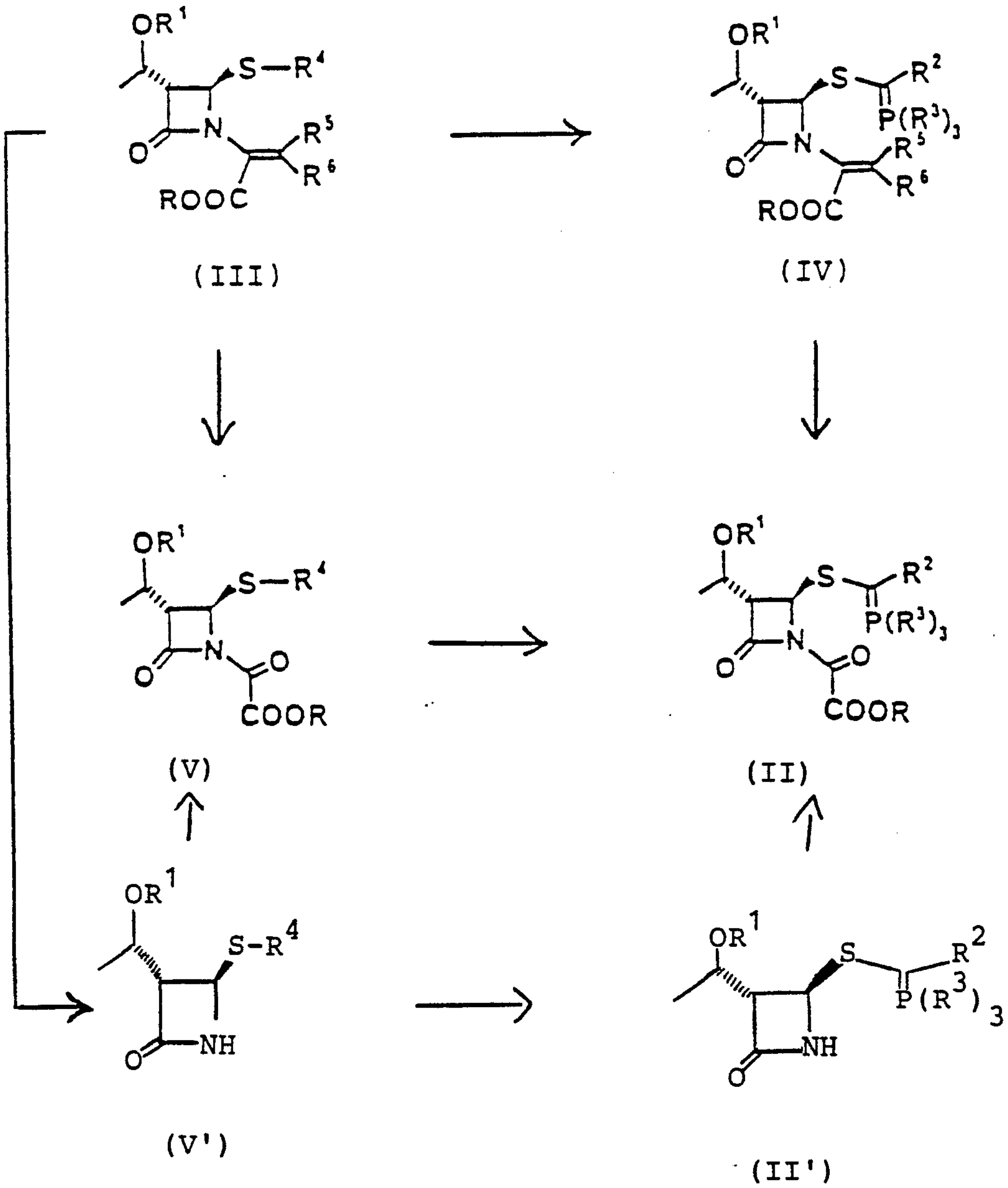

(III) (IV) (V) (II) (V') (II')

in which formulae R and R' are as defined above; each group $R^3$ is defined as above,

$R^4$ represents a pyrrolidin-2.5-dion-1-yl group, a phthalidyl group, a benzenesulphonyl group that may be substituted by one or more methyl groups, or a group $-SR^7$ in which

$R^7$ represents an alkyl group having from 1 to 5 carbon atoms, a phenyl group that is unsubstituted or substituted by one or more substituents selected from chlorine and fluorine atoms and methyl, methoxy and nitro groups, or represents a thiazole or imidazole group which may be fused to a benzene ring, or a thiazole, imidazole or tetrazole group which may have a methyl substituent on a carbon or a nitrogen atom or which may be fully or partially hydrogenated, and

$R^5$ and $R^6$ which may be the same of different, each represents an alkyl group having from 1 to 4 carbon atoms.

In the above formulae the three $R^3$ groups are preferably the same, and are for example alkyl groups having from 2 to 4 carbon atoms, preferably n-butyl groups, or especially phenyl groups optionally substituted by a methyl or methoxy group at the 2-, 3-or 4-position. $R^4$ preferably represents a p-toluenesulphonyl group or a group $-SR^7$ in which $R^7$ is a heterocyclic group, preferably a 2-benzothiazolyl, 4-methylthiazol-2-yl, 1-methyltetrazol-5-yl, 1-methylimidazol-2-yl, 2-benzimidazolyl or 4,5-dihydrothiazol-2-yl group. A 2-benzothiazolyl group is particularly preferred as $R^7$. $R^5$ and $R^6$ preferably both represent methyl groups.

In formulae II', IV, V and V' R' may represent a hydrogen atom or a hydroxy protecting group, but in

formula V and V' when $R^2$ represents an unsubstituted or substituted alkyl, alkoxy, phenyl, phenoxy or phenylthio group, $R^1$ should preferably be a hydroxy protecting group. Any hydroxy group present in a radical $R^2$ should be protected analogously to the 1'-hydroxy group. Amino groups are preferably present in a protecting form as indicated above.

When $R^2$ in compound II represents a -CHO, CN, -CO($C_1$-$C_4$-alkyl) or -COOR$_4$ group, or an optionally substituted alkylthio, alkylsulphinyl, phenylthio or phenylsulphinyl group, compound II may be produced from compound III via compound IV or via compound V. When $R^3$ represents an optionally substituted alkyl, alkoxy, phenyl, or phenoxy group as defined above, the route via compound V is particularly preferred.

Certain compounds of formula III and processes for their production are described in the literature of the art, see, for example, C. Battistini, M. Alpegiani, A. Bedeschi, E. Perrone, C. Scarafile, G. Franceschi; Recent Advances in the Chemistry of $\beta$-lactam Antibiotics, Eds. A.G. Brown and S.M. Roberts, 1984, p. 357 and C. Battistini, C. Scarafile, S. Vioglio, E. Perrone and G. Franceschi, Tet. Lett. 513, 1986. Other compounds of formula III may be prepared analogously. These processes for the production of compound III start from penicillin derivatives that are available commercially, are relatively inexpensive, and already have the appropriate stereochemistry at C-5.

To obtain a compound of formula IV a compound of formula III is reacted with a compound of formula VI

$(R^3)_3P = CHR^2$ (VI)

in which $R^2$ and $R^3$ are defined as above.

As indicated, each group $R^3$ preferably represents an n-butyl, phenyl, tolyl or methoxyphenyl group. In compound III $R^1$ may represent a hydrogen atom or a hydroxy protecting group, $R^4$ preferably represents a p-toluenesulphonyl group or one of the heterocyclicthiol groups $-SR^7$ defined above, especially a 2-benzothiazolylthio group, and $R^5$ and $R^6$ preferably both represent methyl groups.

The reaction between compounds III and VI is generally carried out in a solvent or diluent that is inert under the reaction conditions, for example, acetonitrile, a chlorinated hydrocarbon, for example, chloroform or dichloromethane, an aliphatic or aromatic hydrocarbon, for example, hexane, benzene or toluene, an ether, for example, diethyl ether or tetrahydrofuran, or an ester, for example, ethyl acetate. The reaction temperature is, for example, within the range of from -70 to 60°C; room temperature is preferred. It is preferable to use compounds III and VI in a molar ratio of from 1:1 to 1:3 especially from 1:1 to 1:2.

As indicated above, compound IV may be converted into compound II by oxidation using ozone or another oxidising agent, for example, potassium permanganate, periodic acid or osmium tetroxide-sodium periodate. Ozonolysis is preferred.

During the oxidation the phosphorane group

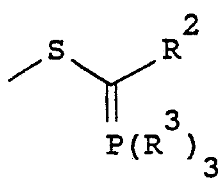

must be protected by conversion into phosphonium salt form. Accordingly, if not already in salt form compound IV is treated with an inorganic or organic acid, for example, hydrochloric acid, sulphuric acid, acetic acid, oxalic acid, p-toluenesulphonic acid or preferably trifluoro acetic acid. The acid is generally used in an equimolar amount or in an excess, for example, a ten-fold molar excess. In certain cases, for example, when using trifluoroacetic acid, the acid can function as a cosolvent for the subsequent oxidation.

Accordingly, protected compound IV is reacted with the oxidising agent, preferably ozone, to give the correspondingly protected compound II. The reaction is generally carried out in a solvent as described above for the reaction of compounds III and VI and preferably in dichloromethane. The reaction temperature is generally within the range of from -78°C to 0°C, preferably -20°C.

When ozone is used as the oxidising agent, it is preferable to treat the reaction product with a reducing agent to decompose any ozonide product present. The reducing agent is, for example, a tri($C_1$-$C_4$)alkyl phosphite, tri($C_4$-$C_8$)alkylphosphine, triphenylphosphine, sodium hydrogen sulphite, zinc metal or, preferably, a di-($C_1$-$C_4$)alkylsulphide, for example, dimethylsulphide.

The protected compound II resulting from the oxidation reaction (and in the case of ozonolysis, after the optional reduction) is treated, if desired, with a base to obtain the desired compound of formula II having a free phosphorane group. The base may be organic or inorganic, for example, sodium hydride, sodium hydroxide, sodium carbonate or sodium bicarbonate, sodamide, potassium t-butoxide, sodium t-amyloxide,

sodium methylsulphinylmethide, lithium hexamethyldisilazide, lithium diisopropylamide, lithium cyclohexyl isopropylamide, or a tertiary amine, for example, triethylamine, diisopropylethylamine or pyridine. The reaction is generally carried out in a solvent, for example, a chlorinated hydrocarbon, for example, dichloro methane: an ether, for example, tetrahydrofuran, 1,2-dimethoxyethane or diethyl ether; an aliphatic or aromatic hydrocarbon, for example, hexane or toluene; acetonitrile; or an amide, for example, N-methylpyrrolidone.

As indicated above, a compound of formula II may be obtained from a compound of formula III via a compound of formula V. According to this route, compound III is oxidised using ozone or another oxidising agent, for example, potassium permanganate, periodic acid or osmium tetroxide-sodium periodate. Again, ozonolysis is preferred. The reaction conditions are generally as described above for the oxidation of compound IV, but lower temperatures within the generally indicated range, for example, from -78°C to 0°C are preferred, for example, -70°C. A solvent is generally used, for example, as described above for the oxidation of compound IV. Further solvents, for example, ketones, for example, acetone may be used.

Compound V is then reacted with a phosphorane of formula VI as described above. When $R^2$ represents a -CN, -COOR, -CHO, -CO($C_1$-$C_4$-alkyl) or an optionally substituted phenylsulphinyl group, the phosporane VI is generally stable and may be obtained and used as such or, if desired, may be produced in situ. When $R^2$ represents an optionally substituted alkyl, alkoxy, alkylthio, alkylsulphinyl, phenyl or phenoxy group, the phosphorane VI is not generally stable to isolation, and should be produced in situ.

A compound of formula VI may be produced by reacting a salt of formula VIa

$(R^3)_3P^{\oplus}$ $-CH_2R^2 \bullet X^{\ominus}$ (VIa)

in which $R^2$ and $R^3$ are defined as above and $X^{\ominus}$ represents a monovalent anion, for example, a halogen ion, especially a chloride, bromide or iodide ion, or, for example, $HSO_4^{\ominus}$, $PF_6^{\ominus}$, $^{\ominus}OCO(C_1$-$C_4$-alkyl), with a base, for example, potassium t-butoxide, sodium hydride, sodium ethoxide, sodium methylsulphinylmethide (dimsyl sodium), n-butyllithium, phenyllithium, or a metallated amine, for example, lithium hexamethyldisilazide, lithium 2,2,6,6-tetramethylpiperidide, lithium cyclohexyl isopropylamide, lithium diisopropylamide and sodamide. Examples of preferred bases are the metallated amines listed above, n-butyllithium, dimsyl sodium and potassium t-butoxide. The base may be used in an amount equimolar to compound VIa or in an excess, for example, a two-fold molar excess.

The compound of formula VIa and the base are generally reacted in a solvent, for example, an ether, for example, diethyl ether, 1,2-dimethoxyethane or, preferably tetrahydrofuran, or a hydrocarbon, for example, hexane, benzene or toluene; acetontrile, dimethylformamide or dichloromethane. The reaction temperature is generally within the range of from -78°C to the boiling point of the solvent used. In the case of optionally substituted alkyl, alkoxy, alkylthio, alkylsulphinyl, phenyl and phenoxy radicals $R^2$, it is preferable to use temperatures in the lower part of the range, for example, from -78°C to -20°C.

A compound of formula VI whether produced in situ or obtained otherwise, is reacted with a compound of formula V to give a compound of formula II. A solvent is generally used. When compound VI is produced in situ the solvent used for its production generally suffices for the subsequent reaction with compound V, although another solvent may be used, if desired. If compound VI is obtained per se solvents as described above for the production of compound VI may be used.

The temperature of the reaction between compound V and VI may be within the range of from -78°C to the boiling point of the solvent used. It is generally convenient to admix a solution of the compound of formula V and the reaction mixture in which compound VI was produced and to allow the resulting reaction mixture to warm to room temperature. If desired, the reaction mixture can be heated to above room temperature, for example, to 80°C to accelerate the reaction, particularly in the case when $R^2$ represents a -CN, -CHO, -COOR or -CO($C_1$-$C_4$-alkyl) group. The phosphorane of formula VI is generally used in an equimolar amount or in a molar excess relative to compound V. A molar ratio compound V:compound VI of 1:3 and especially 1:2 may be used.

In the case of a compound VI in which $R^2$ represents a -CN, -CHO, -COOR or -CO($C_1$-$C_4$-alkyl) group, no base is generally required in the reaction mixture. In the case of other compounds V, the presence of a base is preferred. This may be an excess of compound VI (see above) or another base may be used, for example, an alkoxide having from 1 to 4 carbon atoms, sodium hydride, dimsyl sodium, sodamide, n-butyllithium, lithium hexamethyldisilazide, phenyllithium, lithium diisopropylamide, lithium cyclohexyl isopropylamide or lithium 2,2,6,6-tetramethylpiperidide.

Alternatively compounds V and II may be produced from compounds V′ or II′ by known methods, cf. A. Afonso et al., J. Am. Chem. Soc. 104, 6139 (1982), by acylation with an oxalyl chloride derivative of formula VII

Cl CO COOR (VII)

in which R is defined as above.

The conversion of compounds V' to compounds II' proceeds analogously to the methods described above for the production of IV from III.

Compounds of formula V' may be prepared from compounds III according to known procedures, see, for example, M. Davis, W-Y. Wu, Aust. J. Chem. 40, 223 - 9 (1987).

A compound of formula II, whether produced from compounds IV, V or II', may be isolated, for example, chromatographically, or may be reacted further in situ to give a compound of formula I.

In some cases when the cyclisation of a compound II occurs particularly readily, it may not be possible to isolate that compound, but its presence during the cyclisation reactions can be demonstrated analytically. In general it is advantageous to cyclise compound II in situ, but in some cases, where appropriate, it may be preferable to isolate and/or purify compound II.

In compound I, the group $R^2$ may be the ultimately desired group, or it may be a group that can be converted into a desired group $R^2$. Such reactions are described in more detail below, as are the various reactions that can be used to produce esters and salts of various types of compounds I.

The possibility of modifying a substituent on the penem ring is particularly useful for the production of a compound of formula I having a substituent $R^2$ that is potentially unstable in any of the reactions involved in the production of that compound, or that is incompatible with any of the reagents used. In such a case, $R^2$ may function as a protected form of the desired group, or there may be used a different but stable or compatible group that can be converted into the desired group. The conversion step is, accordingly, carried out after the reaction or reactions in which the desired substituent is potentially unstable or incompatible.

In a compound of formula I, a conversion may be carried out before or after the removal of a protecting group from the 8-hydroxy group, and before or after the removal of a 2-carboxy protecting group, having regard to the potential reactivity of a free hydroxy group and of a free carboxy group in the reaction under consideration.

Examples of such conversions (including conversions of radicals forming a part of a larger group) are the following:

(i) -COOR, $-COOSi(R_a)_3$ and -COO-phenyl to -COOH,

(ii) -COOH to $-CONH_2$, $-CONH(C_1\text{-}C_4\text{-alkyl})$ or $-CONH(CH_2)_mQ$

(iii) -COOH to -COOR

(iv) -COOH to -CO-alkyl

(v) $-NH_2$ to -NHCHO, -NH-CH=NH, $-NHCOR_d$, $-NHSO_2R_d$, $-NHCONHR_d$,

$-NHCONHR_d$, 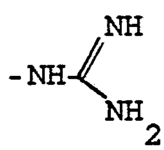

(vi) halogen to -CN or -COOH

(vii) $-S(C_1\text{-}C_4\text{-alkyl})$ to $-SO(C_1\text{-}C_4\text{-alkyl})$ or $-SO_2(C_1\text{-}C_4\text{-alkyl})$

(viii) -CN to $-CH_2NH_2$, which is then optionally converted to a group as defined in (v) above.

(xi) $-SO(C_1\text{-}C_4\text{-alkyl})$ to $-SO_2(C_1\text{-}C_4\text{-alkyl})$

(xiii) $-NO_2$ to $-NH_2$, which may then be converted further as described in (v) above.

The conversions described above may be used in any appropriate combination, for example, conversion of a halogen atom to a nitrile group which may then be converted into an aminomethyl group, which may be reacted further. In some cases, the group that is converted may not be a group $R^2$ itself, but may be a moiety forming part of such a group, for example, an $-NH_2$ group may be a group Q as defined above or the terminal moiety of a group Q.

Many of the methods for carrying out such reactions are known per se in the art, for example,

(i) a carboxy protecting group may be removed by conventional methods, for more details see below;

(ii) a carboxy group may be amidated using an amine and a condensing agent, for example, a carbodiimide, or by reacting an amine with an activated carboxylic acid derivative, for example, an active ester or an anhydride (symmetrical or unsymmetrical), or an acid chloride;

(iii) a carboxylic acid group may be esterified using an alcohol and an activated carboxylic acid derivative, for example, an active ester, acid anhydride or acid chloride;

(iv) a carboxy group may be converted to a ketone by reaction with an alkyllithium compound;

(v) substitution of an amine group by an acyl group or an alkyl or substituted alkyl group as defined in (v) may be carried out conventionally, for example, an amino group may be acylated with, for example, an acid chloride or an acid anhydride, for example, acetyl chloride or acetic anhydride, or with the appropriate acid derivative;

(vi) a halide, especially an iodide, may be converted to a cyanide by treatment with an alkali-metal cyanide and a copper catalyst;

(vii) an alkylthio group may be oxidised, preferably with a carboxylic peracid, especially m-chloroperbenzoic acid, to give the corresponding alkylsulphinyl or alkylsulphonyl group;

(viii) a cyano group may be converted to an amino group by reduction, for example, using a metal hydride, which amino group is then reacted further as described above;

(ix) an alkylsulphinyl group may be oxidised to an alkylsulphonyl group as described in (vii) above;

(x) a nitro group may be reduced to an amino group by noble metal catalysed hydrogenation, for example, using platinum or 10 % palladium on carbon, c.f. M. Freifelder, Catalytic Hydrogenation in Organic Synthesis, Wiley Interscience, 1978, page 26, and P.N. Rylander, Catalytic Hydrogenation over Platinum Metals, Academic Press, 1967, Chapter 11, and the amino group is then reacted further as described in (v) above.

In formulae I and II, R' may represent a hydrogen atom or a hydroxy protecting group. When compound II is obtained from compound IV it is preferable to protect the hydroxy group, and when compound II is obtained from compound V, the hydroxy group should be protected.

It may also be preferable or essential to protect the 2-carboxy group during the formation of a compound of formula I.

Hydroxy protecting groups R' are tetrahydropyranyl and tetrahydrofuranyl groups; $-Si(R_a)_3$ groups in which the three groups $R_a$ are the same or different and each represents an alkyl group having from 1 to 4 carbon atoms or a phenyl group, for example, giving trimethylsilyl, triethylsilyl, diphenyl-t-butylsilyl, dimethyl-t-butylsilyl, and methyldiphenylsilyl groups; and $-COOR_b$ groups in which $R_b$ represents a p-nitrobenzyl, 2,2,2-trichloroethyl, 2-trimemethylsilylethyl, benzyl or allyl group.

Preferred hydroxy protecting groups R' are tetrahydropyranyl, trimethylsilyl, 2-trimethylsilyl, ethoxycarbonyl, p-nitrobenzyloxycarbonyl, allyloxycarbonyl, 2,2,2-trichloroethoxycarbonyl and, especially, triethylsilyl and t-butyldimethylsilyl groups.

Some of these groups may be removed by acid hydrolysis, for example, using 0.1 to 2M, preferably 0.5M hydrochloric acid, for example, tetrahydrofuran, cf. Belgian Patent Specification No. 881 012; n-$Bu_4NF$ in an acidic medium, for example, in an acidic medium, for example, in acetic acid, cf. Belgian Patent Specification No. 882 764; or aqueous hydrogen fluoride, for example, in the presence of acetonitrile, cf. J. Chem. Soc. Perkin 1, 1981, 2055.

In a compound of formula I, the 8-hydroxy group, if esterified, is preferably esterified with a group that can be removed in vivo to give the free hydroxy group, that is to say, an ester group that can be removed under physiological conditions. Examples of suitable esterifying groups are carboxylic acid acyl groups of the formula $-R_cCO$-in which $R_c$ represents a hydrogen atom or a straight or branched chain alkyl group having from 1 to 6 carbon atoms, especially a methyl, ethyl or t-butyl group, or represents a phenyl group or a phenoxyalkyl group in which the alkyl moiety is straight-chained or branched and has from 1 to 4 carbon atoms, and is especially a methyl group.

A non-physiologically removable protecting group R' is generally removed from a resulting compound of formula I and may be replaced by a physiologically removable group, if desired. In some cases, a carboxylic acid acyl group R' may fulfil a protective role during the synthesis of compound I. Such a dual funtion protective group may be removed, retained or replaced in formula I as desired.

An ester group at the 8-position may be the only ester group present, or it may be present in addition to an ester group at the 2-carboxyl group. As mentioned above, a physiologically removable group may be present as a protecting group R' during the formation of a compound of formula I, or it may be introduced at the free 8-hydrody group of a compound of formula I, after removal of a non-physiologically removable hydroxy protecting group, if present. An esterifying group may be introduced at the 8-hydroxy group by a reaction with an organic acid derivative in known manner. A particularly convenient method is to react a compound of formula I with an activated acid derivative, for example, an acid anhydride in the presence of an organic base, for example, 4-dimethylaminopyridine.

As indicated above, a compound of formula I or II may be in the form of an ester at the carboxy group at position 2 in formula I or the corresponding position 2" in formula II.

In an esterified carboxy group -COOR, the group R may be, for example, a straight or brached chain alkyl group having from 1 to 4 carbon atoms, or an alkenyl or a halogen substituted alkenyl group having from 2 to 4 carbon atoms, for example, a methyl, ethyl, n-propyl, iso-propyl, n-butyl, sec-butyl, iso-butyl,

tert.-butyl, allyl, 2-chlorallyl, 3-chlorallyl or vinyl group. R may also represent a benzyl group that is unsubstituted or is substituted by one or two substitutents selected from methoxy and nitro groups and halogen atoms; a 1'-(phenoxy)ethyl group; a 2,2,2-trichloroethyl group; a trialkylsilyl or trialkylsilylalkyl group in which each alkyl moiety has from 1 to 4 carbon atoms; a phthalidyl group, a benzhydryl group or a trityl group, or an acetonyl group. Furthermore R may represent an acyloxymethyl or 1'-(acyloxy)ethyl group having from 2 to 12 carbon atoms in the acyl moiety, an aminoalkanoyloxymethyl or 1'-(aminoalkanoyloxy)-ethyl group having from 2 to 12 carbon atoms in the alkanoyl moiety; an alkoxycarbonyloxymethyl or 1'-(alkoxycarbonyloxy)ethyl group having from 2 to 12 carbon atoms in the alkoxycarbonyl moiety; or an optionally substituted 2-aminoethyl group.

As indicated above, an ester group is especially one that can be removed by hydrolysis, photolysis, oxidation, reduction or enzyme action, or two or more of these methods may be used, for example, reduction followed by hydrolysis. A group R that can be removed readily without substantial degradation of the rest of the molecule is particularly useful as a carboxy protecting group R. Examples of esters that are readily split by reduction are 2,2,2-trichloroethyl esters, and phenyl substituted-methyl esters, which may be unsubstituted or substituted, for example, benzyl, p-nitrobenzyl, benzhydryl and trityl esters.

Reduction of an ester, for example, a phenyl substituted-methyl ester, for example, a p-nitrobenzyl ester, may be carried out using hydrogen an a metal catalyst, for example, a noble metal catalyst, for example, platinum, palladium or rhodium, which catalyst may be supported, for example, on charcoal or kieselguhr. Such reductions may be carried out in the presence of a salt-forming agent, for example, sodium or potassium bicarbonate, if it is desired to form directly a salt at the 2-carboxylic acid group of formula I.

Alternatively, a p-nitrobenzyl ester may be converted into the corresponding free acid by a two-step method, with an initial reduction of the nitro group followed by hydrolysis. The nitro group may be reduced by noble metal catalysed hydrogenation, for example, using platinum, or palladium on carbon, or by a metal reducing agent, for example, zinc in acetic acid. Other metal reducing agents are, for example, aluminum amalgam, and iron and ammonium chloride, see for example, British Patent Specification No. 1,582,960. Reduction of the nitro group is followed by hydrolysis which may occur in situ during reduction of the nitro group or which may be carried out subsequently by treatment with an acid or a base.

Allyl-or halogen-substituted allylester may be cleaved by metal complexes, such as Pd $(PPh_3)_4$, see for example P.D. Jeffrey and S.W. McCombie, J. Org. Chem. 47, 587 (1982). Certain ester groups may be split off by base hydrolysis, for example, acetylmethyl and acetoxymethyl esters.

Some of the ester groups may be cleaved by oxidative hydrolysis, for example, the dimethoxybenzyl group.

Other cleavable esters include the trialkylsilyl and trialkylsilylalkyl esters in whcih alkyl moieties have, independently, from 1 to 4 carbon atoms, for example, trimethylsilylethyl esters.

In the above process, removal of esterifying groups by oxidative and reductive processes may be achieved electrochemically.

There may be used an esterifying group that is removable under physiological conditions, that is to say, the esterifying group is split off in vivo to give the free acid or a carboxylate, for example, an acyloxymethyl or 1'-(acyloxy)ethyl ester having from 2 to 12 carbon atoms in the acyl moiety, for example, an acetoxymethyl, 1'-(acetoxy)ethyl or pivaloyloxymethyl ester, a 5-methyl-2-oxo-1,3-dioxolen-4-yl-methyl ester, an aminoalkanoyloxymethyl ester having from 2 to 12 carbon atoms in the alkanoyl moiety, for example, a glycyloxymethyl, L-valinyloxymethyl or L-leucyloxymethyl ester, or a phthalidyl ester, or a 1'-(alkoxycarbonyloxy)ethyl ester, for example, a 1'-(methoxycarbonyloxy)ethyl or 1'-(ethoxycarbonyloxy)ethyl ester, or an optionally substituted 2-aminoethyl ester, for example, a 2-diethylaminoethyl or 2-(1-morpholino)-ethyl ester.

Preferred esters are the p-nitrobenzyl, p-methoxybenzyl, 2,2,2-trichloroethyl, benzhydryl, 2-trimethylsilylethyl, allyl, 2-chloroallyl, 3-chloroallyl, phthalidyl, pivaloyloxymethyl, ethoxycarbonyloxymethyl, 5-methyl-2-oxo-1,3-dioxolen-4-yl-methyl, acetoxymethyl and 1'-(acetoxy)ethyl esters.

An ester at any position in a compound of formula I or of any other free acid described herein, may be prepared by reaction of the appropriate free acid or activated derivative thereof with an alcohol, a phenol or a reactive derivative thereof. The reaction is preferably carried out under mild conditions in order to prevent rupture of the ring system, for example, under neutral or mild acidic or basic conditions, and at temperatures within the range of from -70 to +35°C.

An ester derived from an alcohol may also be produced by reaction of a reactive derivative of the alcohol, for example, a halide, for example, a chloride, bromide or iodide, or hydrocarbonsulphonyl derivative, for example, a mesyl or tosyl ester, with a salt of an acid of formula I or of another free acid described herein for example, an alkali or alkaline earth metal salt, for example, a lithium, sodium,

potassium, calcium or barium salt, or an amine salt, for example, a triethylammonium salt. The reaction is preferably carried out in a substituted sulphoxide or amide solvent, for example, in dimethyl sulphoxide, dimethylformamide, or hexamethylphosphoramide or, alternatively, an ester may be prepared by reaction of an alcohol or phenol with the acid, for example, in an activated form, for example dicyclohexylcarbodiimide.

The present invention also relates to salts of those compounds of formula I that have salt-forming groups, especially the salts of a free acid of formula I and acid addition salts of compounds of formula I having a basic group. The salts are especially physiologically tolerable salts, for example, alkali metal and alkaline earth metal salts, for example, sodium, potassium, lithium, calcium, and magnesium salts, ammonium salts, and salts with organic amines; also physiologically tolerable acid addition salts. These may be formed with a suitable inorganic or organic acid, for example hydrochloric acid, sulphuric acid, or an organic carboxylic or organic sulphonic acid, for example, p-toluenesulphonic acid.

A salt of a free acid of formula I may be produced by reacting the free acid with the appropriate base in a solvent, preferably under those conditions under which the salt precipitates. A preferred base is potassium 2-ethylhexanoate.

A salt may be produced directly from an ester by splitting off the ester group under suitable reaction conditions, for example, catalytic reduction of an ester, for example, a p-nitrobenzyl ester, in an aqueous organic solvent, for example, comprising water and ethyl acetate, dioxane or tetrahydrofuran, in the presence of a metal salt, especially a metal bicarbonate, for example, in an equivalent amount or in a slight excess, yields the salt directly.

When an acidic centre and a basic centre are both present in a compound of formula I, the compound may exist in zwitterionic form.

Protecting groups may be introduced or removed at any appropriate point in the reactions involved in the production of a compound of formula I.

At any stage in the production of a compound of formula I, a compound produced may be isolated from the reaction mixture in which it was prepared and, if desired, purified by the appropriate techniques used for the preparation of organic compounds, for example, chromatography and crystallisation.

As indicated above, various intermediates may be produced in the form of mixtures of isomers of various kinds. Such mixtures may be separated or resolved at any stage, or an isomeric mixture may be used per se for subsequent reactions.

Any mixture of two or more isomeric forms of a compound of formula I may be resolved, if desired, or a compound of formula I can be used in the form of an isomeric mixture.

Compound I has 5R,6S-stereochemistry and its precursors of formulae II, II', III, IV, V and V' have 3S, 4R-stereochemistry.

At position 8 in formula I the preferred stereochemical configuration is R. If compound I or its precursors are in the form of an isomeric mixture, this may be resolved or separated, if desired, as mentioned above, or in the isomeric mixture may be used. If desired, a compound of formula I, III, IV or V having 8S-or 1'S-sterochemistry, as appropriate, can be subjected to an inversion procedure to give a compound having the desired 8R or 1'R-configuration.

Methods for carrying out a stereochemical inversion at the corresponding 8-position of a penem are known, see, for example, I. Shinkai et al., Tetrahedron Letters, 1982, 4899.

In one example of such a technique a free hydroxy group is oxidised to give an oxo group, which is then reduced to a hydroxy group. The oxidation may be carried out using, for example, a dimethylsulphoxide-trifluoroacetic anhydride-triethylamine reagent, of A.K. Sharma and D. Swern, Tetrahedron Letters, 1974, 1508, and S.L. Huang et al., Synthesis 1983, 297. The reducing agent used may be an alkali metal tri-sec-butylborohydride in which the alkali metal component is, for example, lithium, sodium or potassium, c.f. F.A. Bouffard et al., J. Org. Chem. 1981, 46, 2210, or may be a di-isopropylamine-borane complex in the presence of magnesium trifluoroacetate c.f. P.J. Reider et al., Tetrahedron Letters 23, 2293 (1982). The reduction may give one substantially pure diastereoisomer, or a mixture of isomers which can be separated.

In another method, a di-lower alkyl azodicarboxylate-triphenylphosphine complex reagent is used. This type of reaction is described by O. Misonobu in Synthesis, 1981, page 1. It involves, for example, reacting the free hydroxy compound with a mixture of triphenylphosphine and the di-lower alkyl azodicarboxylate in the presence of a nucleophilic agent, for example, an acid, for example, a carboxylic acid, for example, formic or benzoic acid, or a carboxylate and in solution in a solvent that is inert to the reaction conditions followed by hydrolysis of the modified hydroxy group to give the free hydroxy group again, for example, as shown below in the case of a hydroxy group converted into a benzoyloxy group:

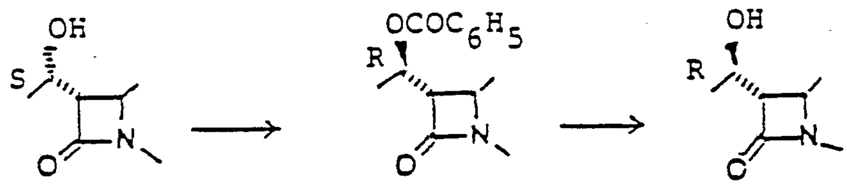

As indicated above, compounds of formula I either have antibacterial activity themselves or are precursors for antibacterially active compounds.

As indicated above, the compounds of formula I and salts thereof are β-lactamase inhibitors, and the compounds are generally stable to the action of β-lactamases produced by gram-positive organisms, for example, Staphylococcus aureus and gram-negative organisms, for example, Enterobacter cloacae. They also possess antibacterial properties themselves and may be used in humans and other animals, for example, to treat bacterial infections caused by gram-positive and gram-negative bacteria, for example, Staphylococcus aureus, Streptococcus pyogenes, Bacillus substillis, Escherichia coli, Pseudomonas aeruginosa and Proteus morganii, some strains of which are penicillin resistant.

The present invention accordingly provides a pharmaceutical preparation which comprises a compound of formula I produced according to the process of the present invention or a physiologically tolerable salt thereof, or a mixture of two or more such substances as active ingredient, in admixture or conjunction with a pharmaceutically suitable carrier. The preparation may also comprise one or more other pharmaceutically active substances, for example, another antibacterial substance, especially one having a β-lactam ring. The preparations may be in a form suitable for enteral or parenteral administration, for example, for oral intravenous or intramuscular administration, for example, as tablets, capsules, syrups, or sterile injection or infusion solutions. The preparations are advantageously in unit dosage form and preferably comprise from 10 to 2000 mg of the active ingredient per unit dose. The daily dosage of the active ingredient is generally from 20 to 8000 mg, in divided doses, generally up to 4 doses, for an adult person of about 75 kg body weight.

The invention also provides the use of a compound of formula I produced accordingly to the process of the present invention or a physiologically tolerable ester or salt thereof for the manufacture of a medicament for the treatment of bacterial infections.

The invention further provides a method of treating mammals, especially humans, to combat a bacterial infection, which comprises administering to the mammal a therapeutically effective amount of a compound of formula I produced according to the process of the present invention or a physiologiclly tolerable ester or salt thereof.

The invention further provides a pharmaceutical preparation which comprises an active ingredient as defined above, in unit dosage form.

The invention also provides a pharmaceutical preparation which comprises an active ingredient as defined above, or a physiologically tolerable salt thereof or a mixture of two or more such substances, and one or more further pharmaceutically active substances, in unit dosage form.

Unit doses are preferably as described above. Compounds of formula I are also useful for the production of other penem derivatives.

Of particular interest are the following compounds of the invention, or an ester thereof at the 8-and or 2-position, or a salt thereof.

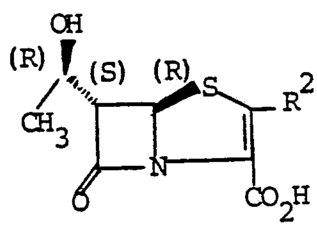

$R^2$ = H, COOH, $CH_2OCONH_2$, $CH_2OCOCH_3$, $CH_2NH_2$, $CH_2CH(CH_3)NH_2$, $SCH_2CH_2OCONH_2$, $SCH_2CH_2OH$, $S-(CH_2)_3SO_2NH_2$, $SCH_2CH_2F$,

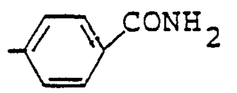
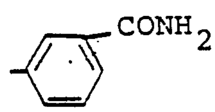
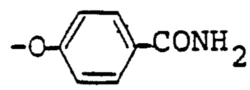
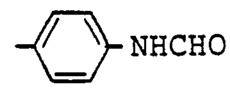

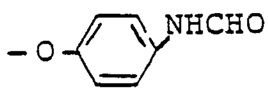
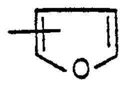
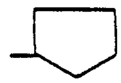
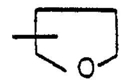

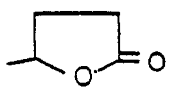
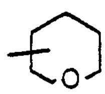
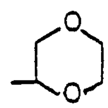

Example 1

{(3(S)-[1(R)-Hydroxyethyl] -1-[ (4-nitrobenzyl)oxycarbonyl-2-methylprop-1-enyl] -2-azetidinon-4(R)-ylthio}-(4-nitrophenyl)-methyltriphenylphosphonium trifluoroacetate

Phenyl-lithium (1.7M in cyclohexane diethyl ether; 1.18 ml) was added to a suspension of 4-nitrobenzyltriphenylphosphonium bromide (0.98 g) in tetrahydrofuran (10 ml) at room temperature. The resultant deep red solution was then allowed to stir for 20 minutes. 5 ml of this solution was then added dropwise during 10 minutes to a briskly stirred solution of 4-nitrobenzyl 2-[4(R)-[benzthiazol-2-yldithio ]-3(S)-[1(R)-hydroxyethyl]-2-oxoazetidin-1-yl} -3-methylbut-2-enoate (0.27 g) in tetrahydrofuran (10 ml) at -72°C. After 20 minutes at -20°C the solvent was evaporated in vacuo and the residual red gum was chromatographed on silica gel. Elution with ethyl acetate isopropanol mixtures gave a red amorphous solid, {3(S)-[1(R)-hydroxyethyl]-1-[(4-nitrobenzyl)oxycarbonyl-2-methylprop-1-enyl]-2-azetidinon-4(R)-ylthio}-(4-nitrophenyl)-methylenetriphenylphosphorane (450 mg), which was converted into the title compound by dissolution in dideutereodichloromethane and addition of an excess of trifluoroacetic acid.
($CD_2Cl_2$ $CF_3CO_2D$): (Major Diastereoisomer) δ 8.24-7.20 (m); 6.35 (1H, d,J = 17.8Hz); 6.18 (1H, d,J = 2.4Hz); 5.16 (2H,s); 4.46 (1H,m); 3.44 (1H,dd,J = 4.4Hz, 2.4Hz); 1.80 (3H,s); 1.77 (3H,s); 1.40 (3H,d,J = 6.5Hz).

Example 2

{3(S)-[1(R)-Hydroxyethyl] -1-[(4-nitrobenzyl)oxycarbonyl-2-methylprop-1-enyl ] -2-azetidinon-4(R)-ylthio } -(phenylthio)-methyltriphenylphosphonium trifluoroacetate

n-Butyl-lithium (1.6M in hexane; 1.14 ml) was added to a suspension of phenylthiomethyltriphenylphosphonium chloride (0.91 g) in dry tetrahydrofuron (10 ml) at room temperature. The resultant yellow solution was then allowed to stir for 20 minutes. 5 ml of this solution was then added dropwise during 5 minutes to a stirred solution of 4-nitrobenzyl 2-{4(R)-[benzthiazol-2-yldithio] -3(S)-[1(R)-hydroxyethyl]-2-oxoazetidin-1-yl} -3-methylbut-2-enoate (0.25 g) in dry tetrahydrofuran (5 ml) at -70°C. After 20 minutes at -20°C the solvent was evaporated and the oil purified on silica gel. Elution with ethyl acetate isopropanol mixtures gave a white foam, { 3(S)-[1(R)hydroxyethyl ] -1-[(4-nitrobenzyl)oxycarbonyl-2-methylprop-1-enyl ] -2-azetidinon-4(R)-ylthio } -(phenylthio)methylenetriphenylphosporane (0.38 g), which was converted into the title compound by dissolution in deuteriochloroform and addition of an excess of trifluoroacetic acid.
($CDCl_3$ $CD_3SOCD_3$ $CF_3CO_2D$): (Major Diastereoisomer)δ 8.07-7.05 (m); 6.33 (1H,d,J = 10.3Hz); 5.62 (1H,d, J = 1.5Hz); 5.24-5.04 (3H,m); 3.20 (1H,dd,J = 4.5,1.5Hz); 2.22 (3H,S); 1.93 (3H,S); 1.33 (3H,d,J = 6.3Hz).

Example 3

Ethyl{3(S)-[1(R)-tert-butyldimethylsilyloxyethyl] -1-[1-methoxycarbonyl-2-methylprop-1-enyl]-2-azetidinon-4-(R)-ylthio}triphenylphosphoranylideneacetate

A solution of ethyl triphenylphosphoranylideneacetate (0.40 g) in dichloromethane (5 ml) was added to a stirred solution of methyl 2-{ 4(R)-[benzthiazol-2-yl-dithio] -3-(S)-[1(R)-tert-butyldimethylsilyloxyethyl]-2-oxoazetidin-1-yl}-3-methylbut-2-enoate (0.620 g) in dichloromethane (15 ml). After 6 hours the solvent was evaporated in vacuo and the residue was chromatographed on silica gel. Elution with hexane/ ethyl acetate mixtures gave the title compound as a white foam.
$\nu$ max ( dichloromethane) 1750, 1716, 1648 $cm^{-1}$
A well resolved proton nmr spectrum could not be obtained for this material.

## Example 4

Tert-Butyl{3(S)-[1(R)-tert-butyldimethylsilyloxyethyl]-1-[ 1-methoxycarbonylmethyloxycarbonyl-2-methylprop-1-enyl ] -2-azetidinon-4(R)-ylthio } triphenylphosphoranylideneacetate

Tert-butyl triphenylphosphoranylideneacetate (0.376 g) was added to a stirred solution of methoxycarbonylmethyl 2-{ 4(R)-[benzthiazol-2-yldithio } -3(S)-[1(R)-tert-butyldimethylsilyloxyethyl]-2-oxoazetidin-1-yl}-3-methylbut-2-enoate (0.300 g) in dichloromethane (10ml).

After 2 hours the solvent was removed in vacuo and the residue was chromatographed on silica gel, deactivated with 10 % of water. Byproducts were eluted with cyclochexane ethyl acetate (5:1), then the title compound with dichloromethane 5% methanol as a white foam (0.300 g)
$\delta(CDCl_3)$ 8.25-7.40 (15H,m); 4.67 (1H,br); 4.64, 4.53 (2H, ABq,J = 16Hz), 4.18 (1H,m); 3.68 (3H,s); 3.17 (1H,br); 2,21 (3H,s); 1.95 (3H,s); 1.63 (3H, br); 0.93 (9H,br); 0.84 (9H,s); 0.05 (3H,s); -0.02 (3H,s).

## Example 5

{3(S)-[1(R)-Tert-butyldimethylsilyloxyethyl] -1-[1-(4-nitrobenzyl)oxycarbonyl-2-methyl-prop-1-enyl] -2-azetidinon-4(R)-ylthio}triphenylphosphoranylideneacetamide

A solution of triphenylphosphoranylideneacetamide (0.23 g) in dichloromethane (10 ml) was added to a stirred solution of 4-nitrobenzyl 2-{4(R)-[benzthiazol-2-yldithio ]-3(S)-[1(R)-tert-butyldimethylsilyloxyethyl ] -2-oxoazetidin-1-yl} -3-methylbut-2-enoate (0.48 g) in dichloromethane (20 ml). After 16 hours the solvent was evaporated in vacuo and the residue was chromatographed on silica gel. Elution with hexane ethyl acetate mixtures gave the title compound as a white foam (0.47 g).
$\nu$ max (dichloromethane 3500, 3390, 1754, 1730, 1650, $cm^{-1}$
A well resolved proton nmr spectrum could not be obtained for this material.

## Example 6

{3(S)-[1(R)-Tert-butyldimethylsilyloxyethyl]-1-[1-methoxycarbonylmethyloxycarbonyl-2-methylprop-1-enyl ] -2-azetidinon-4(R)-ylthio } -(4-chlorophenyloxy)methyltriphenylphosphonium trifluoroacetate

Phenyl-lithium (2M in benzene/diethyl ether; 0.27 ml) was added to a suspension of 4-chlorophenoxymethyltriphenylphosphonium chloride (0.220 g) in tetrahydrofuran (5 ml) at -5°C to obtain a deep red solution. This solution was cooled to -70°C and a solution of methoxycarbonylmethyl 2-{ 4(R)-[benzthiazol-2-yldithio ] -3(S)-[1(R)-tert-butyldimelthylsilyloxyethyl ] -2-oxoazetidin-1-yl} -3-methylbut-2-enoate (0.36 g) in tetrahydrofuran (2 ml), cooled to -60°C, was then added by means of a syringe. After 25 minutes at -20°C, trifluoroacetic acid (0.075 ml) was added, the solvent was evaporated in vacuo and the residue was chromatographed on silica gel, deactivated with 10 % of water. By products were eluted with cyclohexane/ ethyl acetate (1:2), then the title compound with dichloromethane-10 % methanol as a white foam (0.18 g).
$\delta(CDCl_3)$: 7.8-7.1 (20H,m); 5.71 (1H,d,J = 2Hz); 4.62, 4.68 (2H, ABq, J = 16Hz); 4.06 (1H,m); 3.72 (3H,s); 3.12 (1H,dd,J = 2 and 4Hz); 2.12 (3H,s); 1.82 (3H,s); 0.81 (9H,s); 0.70 (3H,d, J = 7Hz); 0.02 (3H,s); -0.08 (3H,s).

The compounds listed in Table 1, which correspond to the general formula IV and carry as radicals R, R', $R^2$ shown in the table and $R^3$ = $C_6H_5$, were obtained as amorphous solids analogously to Example 1

Table 1

| Example | R | $R^1$ | $R^2$ | Characterization |
|---|---|---|---|---|
| 7 | 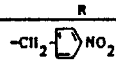 | H | $COCH_3$ | $\gamma_{max}(CH_2Cl_2)$ 1754,1730 $cm^{-1}$ |
| 8 | $-CH_2CCl_3$ | $Si(CH_3)_2\ C(CH_3)_3$ | $COCH_3$ | $\gamma_{max}(CH_2Cl_2)$ 1752,1725 $cm^{-1}$ |
| 9 | $-CH_2CCl_3$ | $CO_2CH_2CCl_3$ | 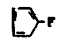 | $\gamma_{max}(CH_2Cl_2)$ 1764,1725 $cm^{-1}$ |
| 10 | $-CH_2CCl_3$ | $Si(CH_3)_2\ C(CH_3)_3$ | 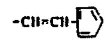 | $\gamma_{max}(CH_2Cl_2)$ 1764,1725 $cm^{-1}$ |
| 11 | 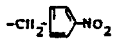 | H | 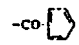 | $\gamma_{max}(CH_2Cl_2)$ 1752,1732 $cm^{-1}$ |
| 12*) | $-CH_2CCl_3$ | $Si(CH_3)_2\ C(CH_3)_3$ | $-SCH_3$ | $\gamma_{max}(CH_2Cl_2)$ 1736m1712 $cm^{-1}$ |
| 13*) | $-CH_2CCl_3$ | $Si(CH_3)_2\ C(CH_3)_3$ | 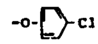 | $\gamma_{max}(CH_2Cl_2)$ 1745,1715 $cm^{-1}$ |

*) prepared analogously to Ex. 6

## Example 14

{3(S)-[1(R)-Hydroxyethyl]-1-[(4-nitrobenzyl)oxycarbonyl-1-oxomethyl] -2-azetidinon-4(R)-ylthio} -4-nitrophenyl) methyltriphenylphosphonium trifluoroacetate

{ 3(R)-[1(R)-Hydroxyethyl]-1-[(4-nitrobenzyl)oxycarbonyl-2-methylprop-1-enyl ] -2-azetidinon-4(R)-ylthio } -(4-nitrophenyl)-methylenetriphenylphosphorane (0.1 g) was dissolved in dideutereodichloromethane (20 ml) and trifluoroacetic acid (0.25 ml) was subsequently added. The resultant solution was then cooled to -20°C before a stream of ozone/oxygen was introduced. After 30 minutes the flow of ozone was stopped and oxygen was passed through the solution for 10 minutes before the solution was allowed to warm to room temperature during 1 hour. A solution of the title compound was thus obtained. Evaporation of the solvent gave a white foam.

($CD_2Cl_2$/$CF_3CO_2D$: (Major Diastereoisomer) δ 8.30-7.17(m), 6.30 (1H,d,J=12.7Hz); 5.38 (2H,ABq); 5.32 (1H,d,J=3.8Hz); 4.28 (1H,m); 3,27 (1H,dd,J=3.9Hz, 3.8Hz); 0.94 (3H,d,J=6.4Hz).

## Example 15

{3(S)-[1(R)-Hydroxyethyl]-1-[(4-nitrobenzyl)oxycarbonyl-1-oxomelthyl ] -2-azetidinon-4(R)-ylthio ] -(phenylthio)-methyltriphenylphosphonium trifluoroacetate

{3(S)-[1(R)-Hydroxyethyl]-1-(4-nitrobenzyl)oxycarbonyl-2-methylprop-1-enyl] -2-azetidinon-4-(R)-ylthio }-(phenylthio)-methylenetriphenylphosphorane (0.13 g) was dissolved in deuteriochloroform (20 ml), and deuteriotrifluoroacetic acid (0.25 ml) was subsequently added. The resultant solution was then cooled to -20 °C before a stream of ozone/oxygen was introduced. After 30 minutes the flow was stopped and oxygen was passed through the solution for 10 minutes before the solution was allowed to warm to room temperature during 1 hour. A solution of the title compound was thus obtained.

($CDCl_3$/$CF_3CO_2D$): (Major Diastereoisomer) δ= 8.28-6.86 (m); 6.45 (1H,d,J=9.4Hz); 5.93 (1H,d,J=1.4HZ); 5.35 (2H,m); 4.38 (1H,m); 3.45 (1H,dd,J=4.5,1.4Hz); 0.90 (1H,d,J=6.4Hz)

## Example 16

Ethyl { 3(S)-[1(R)-tert-butyldimenthylsilyloxyethyl ]-1-[1-methoxycarbonyl-1-oxomethyl ] -2-azetidinon-4(R)-yl-thio}triphenylphosphoranylideneacetate

Trifluoroacetic acid (1 ml) was added to a solution of ethyl { 3(S)-[1(R)-tert-butyldimethylsilyloxyethyl]-1-[1-methoxycarbonyl-2-methylprop-1-enyl ] -2-azetidinon-4(R)-ylthio}-triphenyphosphoranylideneacetate (0.1 g) in dichloromethane (30 ml). The resultant solution was then cooled to -20°C before a stream of ozone/oxygen was introduced. After 40 minutes the flow of ozone was stopped and oxygen was passed

through the solution for 10 mins before adding dimethylsulphide (1 ml). The solution was allowed to warm to room temperature before it was washed with aqueous bicarbonate twice and brine. The solution was then dried over magnesium sulphate and the solvent was removed in vacuo to give the title compound as a pale yellow foam (0.1 g).

νmax (dichloromethane) 1804, 1754, 1706, 1638, 1606 $cm^{-1}$.

A well resolved proton nmr spectrum could not be obtained for this material.

## Example 17

{3(S)-[1(R)-Tert-butyldimethylsilyloxyethyl ]-1-[1-(4-nitrobenzyl)oxycarbonyl-1-oxomethyl]-2-azetidinon-4(R)-ylthio}triphenylphosphoranylideneacetamide

Trifluoroacetic acid (1 ml) was added to a solution of {3(S)-[1(R)-tert-butyldimethylsilyloxyethyl]-1-[1-(4-nitrobenzyl)oxycarbonyl-2-methylprop-1-enyl] -2-azetidinon-4(R)-ylthio } triphenylphosphoranylideneacetamide (0.47 g) in dichloromethane (50 ml). The resultant solution was then cooled to -20 °C before a stream of ozone-oxygen was introduced. After 40 minutes the flow of ozone was stopped and oxygen was passed through the solution for 10 minutes before adding dimethylsulphide (1 ml). The solution was allowed to warm to room tempe-rature before it was washed with aqueous becarbonate twice and brine. The solution was then dried over magnesium sulphate and the solvent was removed in vacuo to give the title compound as a pale yellow foam (0.47 g).

ν max (dichloromethane) 3500, 3390, 1806, 1758, 1702 $cm^{-1}$

A well resolved proton nmr spectrum could not be obtained for this material

## Example 18

{ 3(S)-[1(R)-tert-butyldimethylsilyloxyethyl] -1-[1-methoxycarbonylmethyloxycarbonyl-1-oxomethyl] -2-azetidinon-4(R)-ylthio } -(4-chlorophenyloxy)methyltriphenylphosphonium trifluoroacetate

{3(S)-[1(R)-tert-butyldimethylsilyloxyethyl ]-1-[1-methoxycarbonylmethyloxycarbonyl-2-methylprop-1-enyl] -2-azetidinon-4(R)-ylthio } -(4-chlorophenyloxy)methyltriphenylphosphonium trifluoroacetate (Ex. 6, 0.20 g) was dissolved in dichloromethane (5 ml), methanol (0.068 g) was added and a stream of ozone-oxygen was introduced at - 15 °C during 2 hours. Then dimethylsulphide (0.2 ml) was added and the solution was washed twice with water. The solution was dried over $MgSO_4$ and the solvent was removed in vacuo to give the title compound as a white foam (0.15 g)

δ ($CDCl_3$): 7.75-7.1 (20H, m); 4.90 (1H,d,J=2Hz); 4.78, 4.18 (2H, ABq, J=16Hz); 4.03(1H,m), 3.79(3H, s), 2.78 (1H,dd,J=2 and 4Hz), 1.11 (3H,d,J=6Hz); 0.76 (9H,s); 0.02,-0.05 (two s, 2 x 3H)

The compounds listed in Table 2, which correspond to the general formula II and carry as radicals R, $R^1$, $R^2$, shown in the table and $R^3=C_6H_5$ were obtained as amorphous solids analogously to Example 14

Table 2

| Example | R | $R^1$ | $R^2$ | Cahracterization |
|---|---|---|---|---|
| 19 | $-CH_2$-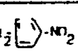-$NO_2$ | H | $COCH_3$ | $\gamma_{max}(CH_2Cl_2)$ 1840,1760,1710 $cm^{-1}$ |
| 20 | $-CH_2CCl_3$ | $Si(CH_3)_2C(CH_3)_3$ | $COCH_3$ | $\gamma_{max}(CH_2Cl_2)$ 1815,1758,1718 $cm^{-1}$ |
| 21 | $-CH_2$-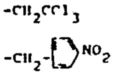$NO_2$ | H | CO-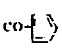 | $\gamma_{max}(CH_2Cl_2)$ 1805,1758,1709 $cm^{-1}$ |

## Example 22

4-Nitrobenzyl 2-{4(R)-[benzthiazol-2-yldithio] -3(S)-[1(R)-tert-butyldimethylsilyloxyethyl ]-2-oxoazetidin-1-yl} -2-oxoacetate

A stream of ozone oxygen was bubbled through a solution of 4-nitrobenzyl 2-{4(R)-[benzthiazol-2-yldithio]-3(S)-[ 1(R)-tert-butyldimethylsilyloxyethyl ]-2-oxoazetidin-1-yl} -3-methylbut-2-enoate (1.31 g) in ethyl acetate (50 ml) for 40 minutes and a stream of oxygen was then bubbled through for 10 minutes. Dimethyl sulphide (3 ml) was then added and the mixture allowed to warm to room temperature before it was washed twice with brine and dried over magnesium sulphate. Removal of the solvent in vacuo gave the title compound as a colourless oil (1.26 g).

$\nu$max (dichloromethane) 1818, 1763, 1709 $cm^{-1}$

$\delta$($CDCl_3$) 8.19-7.33 (8H,m); 5.63 (1H, br.) 5.26 (2H,br.s); 4.36 (1H,m); 3.80 (1H, t, J=2.7Hz); 1.30 (3H, d, J=6.5Hz); 0.82 (9H, s); 0.05 (3H,s); -0.03 (3H,s)

## Example 23

4-Nitrobenzyl 5(R), 3-phenyl-6-(S)-[1(R)-tert-butyldimethylsilyloxyethyl]-7-oxo-4-thia-1-azabicyclo [3.2.0] hept-2-ene-2-carboxylate

n-Butyl-lithium (1.6 M in hexane; 0.16 ml) was added to a suspension of benzyltriphenylphosphonium chloride (0.10 mg) in tetrahydrofuran (10 ml) at -20 °C. After 20 minutes the resultant orange solution was added to a solution of 4-nitrobenzyl 2-{ 4(R)-[benzthioazol-2-yldithio ] -3(S)-[1(R)-tert)-butyldimethylsilyloxyethyl]-2-oxoazetidin-1-yl } -2-oxoacetate (0.165 g) in tetrahydrofuran (10 ml). The resultant mixture was then allowed to stir for 16 hours warming to room temperature before removal of the solvent in vacuo. The residue was chromatographed on silica gel, elution with ethyl acetate/hexane mixtures gave the title compound as a yellow oil (0.05 g).

$\nu_{max}$ ($CDCl_3$) 1781, 1713 $cm^{-1}$

$\delta$($CDCl_3$): 8.13 (2H, part AA′ BB′, J=8.9Hz); 7.56-7.30 (7H,m); 5.72 (1H,d,J=1.6Hz); 5.29, 5.11 (2H,ABq, J=13.8Hz);4.29 (1H,m); 3.81 (1H,dd,J=4.2Hz,1.6Hz); 1.28 (3H,d,J=6.3HZ); 0.86 (9H,s); 0.09 (3H,s); 0.07 (3H,s).

The cyclisation reaction giving the title compound proceeds via { 3(S)-[1(R)-tert-butyldimethylsilyloxyethyl] -1-[(4-nitrobenzyl)oxycarbonyl-2-methylprop-1-enyl]-2-azetidinon-4(R)-ylthio} --(phenyl)-methylenetriphenylphosphorane.

## Example 24

4-Nitrobenzyl 5(R), 3-(phenylthio)-6(S)-[1(R)-tert-butyldimethylsilyloxyethyl]-7-oxo-4-thia-1-azabicyclo[3.2.0]-hept-2-ene-2-carboxylate

n-Butyl-lithium (1.6M in hexane; 0.58 ml) was added to a suspension of phenylthiomethyltriphenylphosphonium chloride (0.39 g) in tetrahydrofuran (20 ml) at - 20°C. After 20 minutes a solution of 4-nitrobenzyl 2-{ 4(R)-[benzthiazol-2-yldithio] -3(S)-[1)R)-tert-butyldimethylsilyloxyethyl] -2-oxoazetidin-1-yl} -2-oxoacetate (0.29 g) in tetrahydrofuran (10 ml) was added. After a further 20 minutes the solvent was evaporated in vacuo and the residue was chromatographed on silica gel. Elution with ethyl acetate/haxane mixtures gave the title compound as a yellow oil (0.145 g).

$\nu_{max}$ (dichloromethane) 1788, 1738, 1730, 1686 $cm^{-1}$

$\delta$($CDCl_3$): 8.22 (2H, part AA′BB′, J=8.7Hz); 7.67-7.37 (7H, m); 5.51 (1H, d, J=1.5Hz); 5.46, 5.27 (2H,ABq, J=13.8Hz); 4.20 (1H, m); 3.60 (1H, dd, J=4Hz, 1.5Hz); 1.15 (3H, d, J=6.3Hz); 0.81 (9H,s); 0.04 (3H,s); 0.02 (3H,s).

The cyclisation reaction giving the title compound proceeds via { 3(S)-[1(R)-tert-butyldimethylsilyloxyethyl)-1-[(4-nitrobenzyl)oxycarbonyl-2-methylprop-1-enyl]-2-azetidinon-4(R)-ylthio}-(phenylthio)-methylenetriphenylphosphorane.

## Example 25

4-Nitrobenzyl 5(R). 3-(4-nitrophenyl)-6(S)-[1(R)-tert-butyldimethylsilyloxyethyl]-7-oxo-4-thia-1-azabicyclo-[3.2.0]hept-2-ene-2-carboxylate

A solution of sodium dimethylsulfinylmethide in dimethylsulphoxide (2.5 ml) freshly prepared from sodium hydride (0.009 g) and dimethylsulphoxide (2.5 ml) was added to a solution of 4-nitrobenzyltriphenylphosphonium bromide (0.158 g) in dimethylsulphoxide (5 ml). After 30 minutes a solution of 4-nitrobenzyl 2-{4(R)-[benzthiazol-2-yldithio] -3(S)-[1(R)-tert-butyldimethylsilyloxyethyl ] -2-oxoazetidin-1-yl } -2-oxoacetate (0.210 g) in dimethylsulphoxide (2 ml) was added. After a further 30 minutes the mixture was partitioned between ethyl acetate and 1m aqueous hydrochloric acid. The phases were separated and the aqueous phase extracted with ethyl acetate. The organic extracts were combined and washed twice with brine and dried over magnesium sulphate. The solvent was evaporated in vacuo and the residue was chromatographed on silica gel. Elution with ethyl acetate/hexane mixtures gave the title compound as a pale yellow oil (0.160 g).

$\nu_{max}$ ($CDCl_3$) 1778 $cm^{-1}$

δ($CDCl_3$: 8.20. 7.62 (4H.AA'BB'. J=8.8Hz); 8.16. 7.51 (4H.AA'BB'. J=8.6Hz); 5.81 (1H,d,J=1.5Hz); 5.30. 5.12. (2H.ABq. J=13.6Hz); 4.29 (1H,m); 3.87 (1H.dd,J=4.0Hz. 1.5Hz); 1.27 (3H,d,J=6.3Hz); 0.86 (9H,s); 0.09 (6H,s).

The cyclisation reaction giving the title compound proceeds via { 3(S)-[1(R)-tert-butyldimethylsilyloxyethyl ] -1-[ (4-nitrobenzyl)oxycarbonyl-2-methylprop-1-enyl] -2-azetidinon-4(R)-ylthio} -(4-nitrophenyl)-methylenetriphenylphosphorane.

## Example 26

4-Nitrobenzyl 5(R). 3-ethoxycarbonyl-6(S)-[1(R)-tert-butyldimethylsilyloxyethyl ] -7-oxo-4-thia-1-azabicyclo-[3.2.0]hept-2-ene-2-carboxylate

Ethyl triphenylphosphoranylideneacetate (0.083 g) was added to a solution of 4-nitrobenzyl 2-{4(R)-[benzthiazol-2-yldithio]-3-(S)-[1(R)-tert-butyldimethylsilyloxyethyl]-2-oxoazetidin-1-yl} -2-oxoacetate (0.15 g) in dichloromethane (40 ml). The solution was then stirred for 16 hours before removal of the solvent in vacuo. The residue was chromatographed on silica gel. Elution with ethyl acetate/mexane mixtures gave the title compound as a colourless oil (0.064 g).

$\nu$max (dichloromethane) 1798, 1738, 1722 $cm^{-1}$

δ($CDCl_3$) 8.20 (2H,d,J=9Hz); 7.57 (2H. d. J=9Hz); 5.75 (1H. d. J=1.8Hz); 5.38. 5.25 (2H. ABq. J=13.5Hz); 4.25 (3H,m); 3.87 (1H. dd,J=3.8Hz. 1.8Hz); 1.28 (3H.t,J=7.3Hz); 1.23 (3H,d. J=7.3Hz); 0.82 (9H,s); 0.06 (6H,s).

The cyclisation reaction giving the title compound proceeds via { 3(S)-[1(R)-tert-butyldimethylsilyloxyethyl] -1-[ (4-nitrobenzyl)oxycarbonyl-2-methylprop-1-enyl]-2-azetidinon-4(R)-ylthio}-4-(ethoxycarbonyl) methylenetriphenylphosphorane.

The compounds listed in Table 3. which correspond to the general formula I and carry as radicals R,R',$R^2$ shown in the table were obtained as amorphous solids from the corresponding compounds V analogously to Example 23

**Table 3**

| Example | R | $R^1$ | $R^2$ | Characterization |
|---|---|---|---|---|
| 27 | $CH_2CCl_3$ | $CO_2CH_2CCl_3$ | - 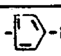 -F | $\nu_{max}$($CH_2Cl_2$) 1810,1750,1722 $cm^{-1}$ |
| 28 | $CH_2CCl_3$ | $Si(CH_3)_2$ $C(CH_3)_3$ | $-SCH_3$ | $\nu_{max}$($CH_2Cl_2$) 1790,1736,1692 $cm^{-1}$ |
| 29 | $CH_2CCl_3$ | $Si(CH_3)_2$ $C(CH_3)_3$ | -CH=CH- 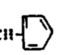 | $\nu_{max}$($CH_2Cl_2$) 1795,1742,1710 $cm^{-1}$ |

## Example 30

4-Nitrobenzyl 5(R), 3-carbamoyl-6(S)-[1(R)-hydroxyethyl]7-oxo-4-thia-1-azabicyclo [3.2.0] hept-2-ene-2-carboxylate

A solution of triphenylphosphoranylideneacetamide (0.16 g) in dichloromethane (5 ml) was added to a solution of 4-nitrobenzyl 2-{4(R)-[benzthiazol-2-yldithio]-3(S)-[1(R)-hydroxyethyl]-2-oxoazetidin-1-yl}-3-methyl-but-2-enoate (0.26 g) in dichloromethane (15 ml). After 3 hours dichloromethane (30 ml) and trifluoroacetic acid (1 ml) were added and the resultant solution was then cooled to -20°C. A stream of ozone oxygen was then bubbled through for 10 minutes. Dimethylsulphide (1 ml) was added and the mixture allowed to warm to room temperature before it was washed twice with aqueous sodium bicarbonate and once with brine. The solution was then dried over magnesium sulphate and the solvent evaporated in vacuo to give a yellow gum. This gum was redissolved in dichloromethane (40 ml) and heated to reflux for 40 minutes and then stood at room temperature for 16 hours. Removal of the solvent gave an off-white foam which was chromatographed on silica gel. Elution with ethyl acetate gave the title compound as a pale yellow solid (0.08 g).

$\nu_{max}$ (dichloromethane) 3450, 1794, 1712, 1670 $cm^{-1}$

δ($CDCl_3$ $(CD_3)_2SO$) 8.22 (3H,m); 7.83 (1H,br,s); 7.68 (2H,d,J = 8.6Hz); 5.75 (1H,d,J = 1Hz); 5.25, 5.32 (2H,ABq, J = 15Hz); 5.18 (1H,br.s); 4.02 (1H,m); 3.94 (1H,dd,J = 5Hz); 1.17 (3H,d,J = 6.2Hz).

## Example 31

Methoxycarbonylmethyl 5(R),3-tert-butoxycarbonyl-6(S)-[1(R)-tert-butyldimethylsilyloxyethyl ] -7-oxo-4-thia-1-azabicyclo[3.2.0]hept-2-ene-2 -carboxylate

Tert-Butyl { 3(S)-[1-(R)-tert-butyldimethylsilyloxyethyl]-1-[1-methoxycarbonylmethyloxycarbonyl-2-methylprop-1-enyl] -2-azetidinon-4(R)-ylthio } triphenylphosphoranylideneacetate (0.258 g, Example 4) was dissolved in dichloromethane (8 ml). Methanol (0.1 ml) and trifluoroacetic acid (0.2 ml) was added and the solution was cooled to -20°C. A stream of ozone/oxygen was bubbled through for 90 minutes. Dimethylsulphide (0.2 ml) was added and the solution was allowed to warm to room temperature. The solution was washed with aqueous sodium bicarbonate and water, dried over $MgSo_4$ and the solvent was evaporated in vacuo to give a white foam, consisting of tert-butyl 3(S)-[1-(R)-tert-butyldimethylsilyloxyethyl] -1-[1-methoxycarbonylmethyloxycarbonyl-1-oxomethyl] -2-azetidinon-4(R)-ylthio} triphenylphosphoranylideneacetate (0.260 g) as a mixture of epimers. This mixture was dissolved in toluene (15 ml) and maintained at 80°C for 3 hours. The solvent was removed in vacuo and the residue was chromatographed on silica gel, deactivated with 10 % of water. Elution with cyclohexanethyl/acetate (5:1) gave the title compound as a colorless, crystalline solid, mp 87°C.

δ($CDCl_3$) 5.71 (1H,d,J = 2Hz); 4.79, 4.67 (2H,ABq, J = 16Hz); 4.23 (1H,m); 3.83 (1H,dd,J = 2 and 4Hz); 3.77 (3H,s); 1.51 (9H,s); 1.24 (3H,d,J = 7Hz); 0.88 (9H,s); 0.08 (6H,s).

## Example 32

Methyl 5(R), 3-ethoxycarbonyl-6(S)-[1(R)-tert-butyldimethylsilyloxyethyl ] -7-oxo-4-thia-1-azabicyclo [3.2.0.] hept-2-ene-2-carboxylate

A solution of ethyl {3(S)-[1(R)-tert-butyldimethylsilyloxyethyl] -1-[1-methoxycarboxyl-1-oxomethyl]-2-azetidinon-4-(R)-ylthio} triphenylphosphoranylideneacetate (0.1 g) in benzene (30 ml) was heated to reflux and maintained at this temperature for 3 hours. The solvent was removed in vacuo and the residue was chromatographed on silica gel. Elution with hexane-ethyl acetate mixtures gave the title compound as a pale yellow oil (0.05 g).

$_{max}$ (dichloromethane) 1798, 1726 $cm^{-1}$

($CDCl_3$ 5.70 (1H,d,J = 1.8Hz); 4.29 (2H,q,J = 7Hz); 4.25 (1H,m,); 3.83 (1H,dd,J = 1.8Hz, 4.3Hz); 3.80 (3H,s); 1.31 (3H,t,J = 7Hz); 1.22 (3H,d,J = 6.3Hz); 0.88 (9H,s); 0.06 (6H,s).

## Example 33:

Methyl 5(R), 3-(4-Chlorophenyloxy)-6(S)-[1(R)-tert-butyldimethylsilyloxyethyl]-7-oxo-4-thia-1-azabicyclo-[3.2.0]hept-2-ene-2-carboxylate

was analogously prepared to example 32 from the compound of example 18 and obtained as a white foam.
$\nu_{max}$ ($CH_2Cl_2$) 1795, 1735 $cm^{-1}$
A well resolved proton NMR spectrum could not be obtained for this material

## Example 34

4-Nitrobenzyl 5(R), 3-Benzoyl-6(S)-[1(R)-hydroxyethyl]-7-oxo-4-thia-1-azabicyclo [3.2.0] hept-2-ene-2-carboxylate

was analogously prepared to Example 32 from the compound of example 21 and obtained as a white foam
$\nu$ max ($CH_2Cl_2$) 3600, 1800, 1756, 1730, 1674 $cm^{-1}$
A well resolved proton NMR spectrum could not be obtained for this material.

## Example 35

4-Nitrobenzyl 5(R), 3-Acetyl-6(S)-[1(R)-hydroxyethyl]-7-oxo-4-thia-1-azabicyclo [3.2.0]hept-2-ene-2-carboxylate

was analogously prepared to Example 32 from the compound of example 19 and obtained as a gum.
$\nu$ max ($CH_2Cl_2$) 1798, 1716 $cm^{-1}$
$\delta$($CDCl_3$ :8.23 (2H,d); 7.58 (2H,d); 5.76 (2H,d); 5.42, 5.26 (2H,ABq); 4.27 (2H,m); 3.90 (1H,dd); 2.44 (3H,s); 1.37 (3H,d).

## Example 36:

4-Nitrobenzyl 5(R), 3-carbamoyl-6(S)-[1(R)-tert-butyldimethylsilyloxyethyl] -7-oxo-4-thia-1-azabicyclo [3.2.0] hept-2-ene-2-carboxylate

A solution of {3(S)-[1(R)-tert-butyldimethylsilyloxyethyl]-1-[1-(4-nitrobenzyl)oxycarbonyl-1-oxomethyl]-2-azetidinon-4(R)-ylthio } triphenylphosphoranylideneacetamide (0.47 g) in dichloromethane (30 ml) was stirred for 16 hours at room temperature. The solvent was removed in vacuo and the residue was chromatographed on silica gel. Elution with hexane-ethyl acetate mixtures gave the title compound as a white solid (0.14 g).
$\nu_{max}$ (dichloromethane) 3464, 1794, 1702, 1674 $cm^{-1}$
$\delta$($CDCl_3$) 9.39 (1H,br.s); 8.23 (2H,d,J=8.7Hz); 7.64 (2H, d, J=8.7Hz); 5.79 (1H,br.s); 5.52 (1H,d,J= 1.8Hz); 5.46-5.23 (2H,AB); 4.24 (1H,m); 3.84 (1H,dd,J=5.1Hz, 1.8Hz); 1.22 (3H,d,J=6.3Hz); 0.79 (9H,s); 0.05 (3H,s); -0.02 (3H,s).

## Example 37

4-Nitrobenzyl 5(R), 3-carbamoyl-6(S)-[1(R)-hydroxyethyl] -7-oxo-4-thia-1-azabicyclo [3.2.0] hept-2-ene-2-carboxylate

To a stirred solution of 4-nitrobenzyl 5(R), 3-carbamoyl-6(S)-[1-(R)-tert-butyldimethylsilyloxyethyl ] -7-oxo-4-thia-1-azabicyclo [3.2.0] hept-2-ene-2-carboxylate (0.14 g) in dry tetrahydrofuran (5 ml) was added glacial acetic acid (0.16 ml) and a 1M solution of tetra-n-butylammonium fluoride in tetrahydrofuran (0.84 ml). After the mixtures had been stirred for 16 hours it was partitioned between ethyl acetate and water; the organic layer was successively washed with aqueous sodium bicarbonate, with water, with brine and was then dried over magnesium sulphate and the solvent evaporated in vacuo. Chromatography of the crude

residue over silica gel, and elution with ethyl acetate-hexane mixtures afforded the title compound as a yellow solid (0.060 g).
$\nu_{max}$ (dichloromethane) 3450, 1794, 1712, 1670 $cm^{-1}$
$\delta$($CDCl_3$,$(CD_3)_2SO$) 8.22 (3H,m); 7.83 (1H,br.s); 7.68 (2H,d,J = 8.6Hz); 5.75 (1H,d,J = 1Hz); 5.25, 5.32 (2H,ABq,J = 15Hz); 5.18 (1H,br.s); 4.02 (1H,m); 3.94 (1H,dd,J = 5Hz, 1Hz); 1.17 (3H,d,J = 6.2Hz)

**Example 38**

4-Nitrobenzyl 5(R), 3-phenyl-6(S)-[1(R)-hydroxyethyl]-7-oxo-4-thia-1-azabicyclo[3.2.0]hept-2-ene-2-carboxylate

To a stirred solution of 4-nitrobenzyl 5(R), 3-phenyl-6(S)-[1(R)-tert-butyldimethylsilyloxyethyl] -7-oxo-4-thia-1-azabicyclo [3.2.0] hept-2-ene-2-carboxylate (0.05 g) in dry tetrahydrofuran (5 ml) was added glacial acetic acid (0.05 ml) and a 1M solution of tetra-n-butylammonium fluoride in tetrahydrofuran (0.25 ml). After the mixture had been stirred for 16 hours it was partitioned between ethyl acetate and water; the organic layer was successively washed with aqueous sodium bicarbonate, with water, with brine and was then dried over magnesium sulphate and the solvent evaporated in vacuo. Chromatography of the crude residue over silica gel, and elution with ethyl acetate-hexane mixtures afforded the title compound as a yellow solid (0.025 g).
$\nu_{max}$ 3450, 1780(sh), 1765, 1695 $cm^{-1}$
$\delta$($CDCl_3$) : 8.12 (2H,part AA'BB', J = 8.7Hz), 7.46 - 7.28 (6H,m); 5.74 (1H,d,J = 1.6Hz); 5.30, 5.12 (2H,ABq,J = 13.9Hz); 4.31 (1H,m); 3.85 (1H,dd,J = 6.5Hz, 1.6Hz); 1.87 (1H,d,J = 4.8Hz); 1.41 (3H,d,J = 6Hz)

**Example 39**

Potassium 5(R), 3-phenyl-6(S)-[1-(R)-hydroxyethyl] -7-oxo-4-thia-1-azabicyclo[3.2.0.]hept-2-ene-2-carboxylate

4-Nitrobenzyl 5(R), 3-phenyl-6(S)-[1(R)-hydroxyethyl]-1-oxo-4-thia-1-azabicyclo [3.2.0] hept-2-ene-2-carboxylate (0.025 g) was dissolved in dioxane (5 ml) and mixed with a solution of potassium hydrogen carbonate (0.006 g) in water (5 ml). The mixture was hydrogenated at 375 kPa (4 atm) over 10 % palladium-on-charcoal (0.025 g) for 1 hour then filtered through Hyflo ( Hyflo is a Trade Mark). The filtrate was freeze-dried. The residue was dissolved in water, washed with ethyl acetate and freeze-dried to give the title compound as a pale yellow powder (0.018 g).
$\delta$($D_2O$): 7.43 (5H,br.s); 5.79 (1H,d,J = 1.5Hz); 4.28 (1H,m); 3.99 (1H,dd,J = 6.1Hz, 1.5Hz); 1.33 (3H,d, J = 6.5Hz).

**Example 40**

4-Nitrobenzyl 5(R), 3-(phenylthio)-6(S)-[1(R)-hydroxyethyl] -7-oxo-4-thia-1-azabicyclo [3.2.0] hept-2-ene-2-carboxylate

The title compound (0.075 g) was obtained by a procedure analogous to that used in Example 38 by using 4-nitrobenzyl 5(R), 3-(phenylthio)-6(S)-[1(R)-tert-butyldimethylsilyloxyethyl]-7-oxo-4-thia-1-azabicyclo-[3.2.0]hept-2-ene-2-carboxylate (0.145 g), glacial acetic acid (0.148 ml), tetrahydrofuran (5 ml) and 1M tetra-n-butyl ammonium fluoride in tetrahydrofuran (0.74 ml).
$\nu_{max}$ (film) 1787, 1690 $cm^{-1}$
$\delta$($CDCl_3$): 8.35-7.25 (9H,m); 5.51 (1H,d,J = 1.2Hz); 5.22, 5.55 (2H,ABq,J = 14.4Hz); 4.20 (1H,m); 3.63 (1H,dd,J = 6.0Hz, 1.2Hz); 2.32 (1H,s); 1.29 (3H,d,J = 6.6Hz).

**Example 41**

Potassium 5(R), 3-(phenylthio)-6(S)-[1(R)-hydroxyethyl]-7-oxo-4-thia-1-azabicyclo[3.2.0] hept-2-ene-2-carboxylate

The title compound (0.024 g) was obtained by a procedure analogous to that used in Example 39 by using 4-nitrobenzyl 5(R), 3-(phenylthio)-6(S)-[1(R)-hydroxyethyl ] -7-oxo-4-thia-1-azabicyclo [3.2.0] hept-2-ene-2-carboxylate (0.066 g), 10 % palladium on charcoal (0.066 g), potassium bicarbonate (0.014 g) water (5 ml) and dioxane (5 ml).
δ($D_2O$): 7.67-7.40 (5H,m); 5.51 (1H,d,J = 1.3Hz); 4.19 (1H,m); 3.77 (1H,dd,J = 5.9Hz, 1.3Hz); 1.23 (3H,d,J = 6.4Hz).

**Example 42**

4-Nitrobenzyl 5(R), 3-(4-nitrophenyl)-6(S)-[1(R)-hydroxyethyl] -7-oxo-4-thia-1-azabicyclo[3.2.0]hept-2-ene-2-carboxylate

The title compound (0.085 g) was obtained by a procedure analogous to that described in Example 38 by using 4-nitrobenzyl 5(R), 3-(4-nitrophenyl)-6(S)-[1-(R)-tert-butyldimethylsilyloxyethyl] -7-oxo-4-thia-1-azabicyclo [3.2.0] hept-2-ene-2-carboxylate (0.160 g), glacial acetic acid (0.163 ml), tetrahydrofuran (5 ml), and 1M tetra-n-butylammonium fluoride in tetrahydrofuran (0.82 ml).
$\nu_{max}$ (film) 1790, 1712 cm⁻¹
δ($CDCl_3$): 8.21, 7.58 (4H,AA'BB,J = 8.9Hz); 8.17, 7.47 (4H,AA'BB,J = 8.7Hz); 5.82 (1H,d,J = 1.6Hz); 5.31, 5.12 (2H,ABq, J = 13.5Hz); 4.30 (1H,m); 3.91 (1H,dd,J = 6.4Hz, 1.6Hz); 1.61 (1H,br.s); 1.41 (3H,d,J = 6.3Hz)

**Example 43**

Potassium 5(R), 3-(4-aminophenyl)-6(S)-[1(R)-hydroxyethyl] -7-oxo-4-thia-1-azabicyclo[3.2.0] hept-2-ene-2-carboxylate

The title compound (0.037 g) was obtained by a procedure analogous to that described in Example 39 by using 4-nitrobenzyl 5(R), 3-(4-nitrophenyl)-6(S)-[1(R)-hydroxyethyl] -7-oxo-4-thia-1-azabicyclo[3.2.0] hept-2-ene-2-carboxylate (0.085 g), 10 % palladium on charcoal (0.085 g), potassium bicarbonate (0.018 g), water (5 ml) and dioxane (5 ml).
δ($D_2O$): 7.30, 6.80 (4H,AA'BB', J = 8.4Hz); 5.70 (1H,d, J = 1.4Hz); 4.23 (1H,m); 3.93 (1H,dd,J = 5.3Hz, 1.4Hz); 1.33 (3H,d,J = 6.3Hz).

**Example 44**

Tert-butyl { 3(S)-[1-(R)-tert-butyldimethylsilyloxyethyl]-1-[1-methoxycarbonylmethyloxycarbonyl-1-oxo-methyl]-2-azetidinon-4(R)-yl-thio} triphenylphosphoranylideneacetate

Method A: **(V'→V→II)**

4(R)-[Benzthiazol-2-yl-dithio]-3(S)-[1(R)-tert-butyldimethylsilyloxyethyl] -2-azetidinone (2.34 g) and allyloxyoxalylchloride (1.64 g) in dichloromethane (15 ml) was treated with diisopropylethylamine (4.1 ml) at 0°C. After 1 hour the solution was washed with 1 N HC1 and with water. The solvent was removed and the residue was chromatographed on silica gel. Elution with toluene:ethyl acetate (10:1) gave allyl 2-{4(R)-[benzthiazol-2-yl-dithio]-3(S)-[1(R)-tert-butyldimethylsilyloxyethyl] -2-oxoazetidin-1-yl}-2-oxoacetate as an oil.
δ($CDCl_3$): 7.95-7.30 (4H, m); 6.05-5.85 (1H, m); 5.68 (1H, br.); 5.46-5.38 (2H, m); 4.34-4.25 (2H, m); 4.35 (1H, m); 3.80 (1H, dd) 1.28 (3H, d, J = 6.5Hz); 0.83 (9H, s); 0.02 and 0.08 (2x 3H, s)

This compound was converted into the title compound using tert-butyl triphenylphosphoranylideneacetate analogously to the procedure described in example 4 and obtained as a white foam.
δ($CDCl_3$): 8.25-7.40 (15H, m); 6.1-5.9 (1H, m); 5.46-5.36 (2H, m); 4.72 (1H, br.); 4.35-4.28 (2H, m); 4.22 (1H, m); 3.33 (1H, br.); 1.55 (3H, br.); 0.95 (9H, br); 0.82 (9H, s); 0.05 (3H, s); - 0.02 (3H, s)

Method B: ($V' \rightarrow II' \rightarrow II$)

Tert-butyl triphenylphosphoranylideneacetate (0.376 g) was added to a solution of 4(R)-[benzthiazol-2-yl-dithio] -3(S)-[1(R)-tert-butyldimethylsilyloxyethyl] -2-azetidinone (0.638 g) in dichloromethane (10 ml). After 3 hours the solvent was removed in vacuo and the residue was chromatographed on silica gel. The product, tert-butyl {3(S)-[1(R)-tert-butyldimethylsilyloxyethyl] -2-azetidinon-4(R)-yl-thio} triphenylphosphoranylideneacetate, was eluted with dichloromethane / 5 % methanol as a white foam (0.42 g).
$\delta(CDCl_3)$: 8.2-7.5 (15H, m); 6.30 (1H, br.); 5.05 (1H, br.); 4.20 (1H, m); 3.25 (1H, br); 1.35 (3H, br.); 0.95 (9H, br.); 0.82 (9H, s) 0.08 (3H, s); 0.02 (3H,s)

This product was converted into the title compound using allyloxyoxalylchlorid and diisopropylethylamine as described above. It was identical in all its properties with the product obtained according to method A.

## Claims

1. A process for the production of a penem compound of formula I or a salt of a penem compound of formula I having a salt forming group

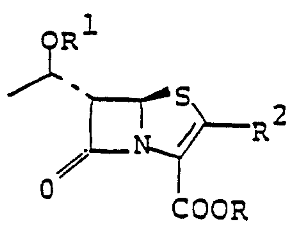

(I)

wherein
R is hydrogen
$C_1$-$C_4$-alkyl, $C_1$-$C_4$-alkyloxycarbonyl-$C_1$-$C_4$-alkyl, $C_2$-$C_4$-alkenyl, that may be substituted by halogen, benzyl that is unsubstituted or is substituted by one or two substituents selected from methoxy, nitro groups and halogen atoms;
1'-(phenoxy)ethyl, 2,2,2-trichloroethyl, $C_1$-$C_4$-trialkylsilyl, $C_1$-$C_4$-trialkylsilyl-$C_1$-$C_4$-alkyl, phthalidyl, benzhydryl, trityl, acetonyl, $C_2$-$C_{12}$-acyloxymethyl, 1'-($C_2$-$C_{12}$-acyloxy) ethyl, amino-$C_2$-$C_{12}$-alkanoyloxy-methyl, 1'-(amino-$C_2$-$C_{12}$-alkanoyloxy) ethyl, $C_2$-$C_{12}$-alkoxycarbonyloxymethyl, 1'-($C_2$-$C_{12}$-alkoxycarbonyloxy) ethyl, optionally substituted 2-aminoethyl,
R' is hydrogen,
tetrahydropyranyl, tetrahydrofuranyl, $Si(Ra)_3$ in which the three groups Ra are the same or different and each represents a $C_1$-$C_4$-alkyl group or a phenyl group;
$CO_2R_b$, in which $R_b$ represents a p-nitrobenzyl, 2-trimethylsilylethyl, 2,2,2-trichloroethyl, benzyl or allyl group;
$COR_c$, in which $R_c$ represents hydrogen, a $C_1$-$C_6$-alkyl group, a phenyl or a phenoxy-$C_1$-$C_4$-alkyl group,
$R^2$ is
hydrogen, -CHO, CN
COOR, in which R is defined as above, CO-$C_1$-$C_4$-alkyl, $C_1$-$C_4$-alkyl, $C_1$-$C_4$-alkyloxy, $C_1$-$C_4$-alkylthio, $C_1$-$C_4$-alkylsulphinyl, $C_1$-$C_4$-alkylsulfonyl, wherein the alkyl groups are unsubstituted or substituted by one or more substituents selected from F, Cl, BR, J atoms; optionally substituted $C_3$-$C_8$-cycloalkyl, phenyl or heterocyclyl; CN, $CO_2R$, $CONH_2$, CONH Rd, CONH($C_1$-$C_4$-alkyl), OH, O($C_1$-$C_4$-alkyl), O($C_3$-$C_8$-cycloalkyl), O-phenyl, O-heterocyclyl, OR', OCO($C_1$-$C_4$-alkyl), $OCONH_2$, OCONH($C_1$-$C_4$-alkyl), $-OCONHR_D$, $S(O)_p$ ($C_1$-$C_4$-alkyl), $S(O)_p$($C_3$-$C_8$-cyclo-alkyl), $S(O)_p$phenyl, $S(O)_p$heterocyclyl, $SO_2NH_2$, $SO_2NHR_D$, $NH_2$, NH ($C_1$-$C_4$-alkyl), N-($C_1$-$C_4$-alkyl)$_2$, NH-phenyl, $NHR_D$, optionally substituted amidino, aminomethylenamino or guanidino, in which groups R and R' are defined as above, $R_D$ represents an amino protecting group and represents 0, 1 or 2;
CO-phenyl, CO-heterocyclyl, phenyl, phenoxy, phenyl-$S(O)_p$, heterocyclyl, heterocyclyloxy, heterocyclyl-S-$(O)_p$, wherein p is defined as above and the heterocycle is a mono-or bicyclic ring system, optionally unsaturated and containing one or more O, S or N atoms and which may be bound via a ring C or N atoms and which may be bound via a ring C or N atom and wherein
the phenyl or heterocyclyl group is unsubstituted or substituted by one or more substituents selected from halogen atoms, $C_1$-$C_4$-alkyl, $C_1$-$C_4$-alkoxy, optionally substituted $C_3$-$C_8$-cycloalkyl and $C_3$-$C_8$-cycloalkyloxy;

$NO_2$, CN, CHO, $CO_2R$, CO($C_1$-$C_4$-alkyl), $CONH_2$, COHN ($C_1$-$C_4$-alkyl), $CONHR_D$, CONHOH, CONHO($C_1$-$C_4$-alkyl), CH = NOH, $CSNH_2$, C( = NOH)$CH_3$, $NH_2$, NH($C_1$-$C_4$-alkyl), $NHR_d$, NHCHO, NHCO($C_1$-$C_4$-alkyl), $S(O)_p$($C_1$-$C_4$-alkyl), $SO_2NH_2$, $SO_2NH$($C_1$-$C_4$-alkyl), $SO_2NHR_D$, $NHSO_2$($C_1$-$C_4$-alkyl), NHCONH($C_1$-$C_4$-alkyl), amidino, aminomethylenamino, guanidino, COX phenyl, in which X is oxygen or sulphur and the phenyl moiety is unsubstituted or substituted by one or more groups, selected from $C_1$-$C_4$-alkoxy, CN, $NO_2$ or halogen atoms, $CO_2Si(Ra)_3$ in which Ra is defined as above, $CONH(CH_2)_mQ$ or -NHCO $(CH_2)_mQ$ in which m represents an integer from 1 to 3, and Q represents one of the following groups:
CN, $SO_2NH_2$, $SO_2NHR_D$, $SOCH_3$, $SOC_2H_5$, $SO_2CH_3$, $SO_2C_2H_5$, $NH_2$, NHCH = NH, $CONH_2$, $CONHR_D$ $CONHCH_3$, CONHOH, NH($C_1$-$C_4$-alkyl), $NHR_D$, $OCONH_2$, NH-C($CH_3$) = NH;
$CONH_2$, CONH($C_1$-$C_4$-alkyl), CONH-phenyl, CH = $CH_2$, CH = CH($C_1$-$C_4$-alkyl), CH = CH-phenyl, CH = CHCO($C_1$-$C_4$-alkyl), CH = CHCO-phenyl or CH = $CHCO_2$($C_1$-$C_4$-alkyl); optionally substituted $C_3$-$C_8$-cycloalkyl, $C_3$-$C_8$-cycloalkyloxy and $C_3$-$C_8$cycloalkyl $S(O)_p$, wherein p and $R_D$ are defined as above and the cycloalkyl group is saturated or contains a double bond and in which one ring carbon atom can be replaced by O, S or N, wherein the cycloalkyl group may be bound via a ring C or N atom and wherein the cycloalkyl group is unsubstituted or substituted as defined above for the alkyl moiety
which process comprises causing or allowing a compound or formula II

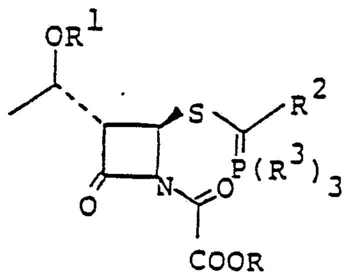

II

in which R, R' and $R^2$ are defined as above and
$R^3$ is $C_2$-$C_8$-alkyl, $C_3$-$C_8$-cycloalkyl or phenyl that is unsubstituted or is substituted by a methyl or methoxy group, the three groups $R^3$ being the same or different, to cyclise.

2. A process as claimed in claim 1 which comprises reacting a compound of formula IV

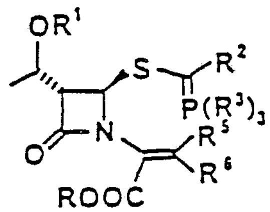

(IV)

in which R, R', $R^2$ and $R^3$ are defined as in claim 1 and $R^5$ and $R^6$, which may be the same or different, each represents an alkyl group having from 1 to 4 carbon atoms, in the form of a phosphonium salt, with ozone or another oxidising agent; and subsequently causing or allowing the formed compound II by treatment with a base to cyclise to give compound I.

3. A process as claimed in claim 1 which comprises reacting a compound of formula V

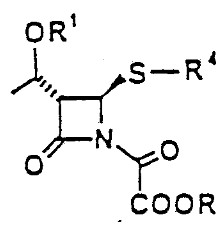

(V)

in which R and R' are defined as in claim 1 and
$R^4$ represents a pyrrolidin-2,5-dion-1-yl group, a phthalidyl group, a benzenesulphonyl group that may be substituted by one or more methyl groups, or a group $-SR^7$ in which

$R^1$ represents an alkyl group having from 1 to 5 carbon atoms, a phenyl group that is unsubstituted or substituted by one or more substituents selected from chlorine and fluorine atoms and methyl, methoxy and nitro groups, or represents a thiazole or imidazole group which may be fused to a benzene ring, especially a 2-benzothiazole group, or a thiazole, imidazole or tetrazole group which may have a methyl substituent on a carbon or a nitrogen atom or which may be fully or partially hydrogenated,
with a compound of formula VI
$(R^3)_3P = CHR^2$ (VI)
in which $R^2$ and $R^3$ are defined as in claim 1.

4. A process according to claim 1 which is carried out under an inert atmosphere.

5. A process according to claim 1 which is carried out in the presence of a free radical acceptor.

6. A process according to claim 1 which is carried out in a solvent.

7. A process according to claim 1 which is carried out within a range of from -70°C to the boiling point of the solvent used.

8. A process according to claim 1 which is carried out within a range of from -20°C to room temperature.

9. A process according to claim 1 which comprises treating a compound of formula II'

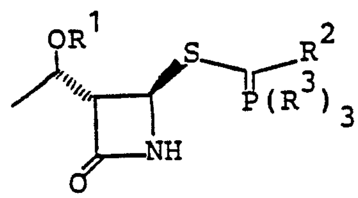

(II')

in which $R^1$, $R^2$ and $R^3$ are as defined above with ClCOCOOR (VII) and subsequently causing or allowing the formed compound II to cyclise.

European Patent Office

# EUROPEAN SEARCH REPORT

Application Number

EP 88 10 0008

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | TETRAHEDRON LETTERS, vol. 25, no. 49, 1984, pages 5593-5596, Pergamon Press Ltd, GB; C.U. KIM et al.: "Carbon nucleophilic displacements on Kamiya's azetidinone disulfide: synthesis of C-2 modified penicillins" * Whole article, especially page 5594, formulas 17,18 * --- | 1-9 | C 07 D 499/00 // C 07 F 9/65 C 07 F 7/18 |
| A | GB-A-2 042 515 (BRISTOL-MYERS) * Pages 19,20,55,56; claims * --- | 1 | |
| A | DE-A-3 213 264 (OTSUKA K.K.) * Claims * ----- | 1 | |
| | | | TECHNICAL FIELDS SEARCHED (Int. Cl.4) |
| | | | C 07 D 499/00 C 07 F 9/00 |

The present search report has been drawn up for all claims

| Place of search | Date of completion of the search | Examiner |
|---|---|---|
| THE HAGUE | 22-03-1988 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document